(19) Europäisches Patentamt
European Patent Office
Office européen des brevets

(11) EP 4 560 641 A1

(12) EUROPEAN PATENT APPLICATION
published in accordance with Art. 153(4) EPC

(43) Date of publication:
28.05.2025 Bulletin 2025/22

(51) International Patent Classification (IPC):
G16H 20/30 (2018.01)        G16H 50/30 (2018.01)
G16H 70/20 (2018.01)        G06F 16/9535 (2019.01)

(21) Application number: 23864663.2

(52) Cooperative Patent Classification (CPC):
A61B 5/00; G06F 16/9537; G06F 16/9538;
G16H 20/30; G16H 50/30; G06F 16/9535;
G16H 70/20

(22) Date of filing: 11.09.2023

(86) International application number:
PCT/CN2023/118017

(87) International publication number:
WO 2024/055931 (21.03.2024 Gazette 2024/12)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 14.09.2022 CN 202211116627

(71) Applicant: Anhui Huami Health Technology Co.,
Ltd.
Hefei, Anhui 230088 (CN)

(72) Inventors:
• SHI, Mingshu
  Hefei, Anhui 230088 (CN)
• WANG, Kongqiao
  Hefei, Anhui 230088 (CN)
• TANG, Mengfan
  Cupertino, 95014 (US)
• GAO, Yi
  Hefei, Anhui 230088 (CN)

(74) Representative: Dai, Simin
Reyda IP
A073
157, Quai du Président Roosevelt
92130 Issy-les-Moulineaux (FR)

(54) EXERCISE RECOMMENDATION METHOD AND APPARATUS, SLEEP RECOMMENDATION METHOD AND APPARATUS, ELECTRONIC DEVICE, AND STORAGE MEDIUM

(57) The present application provides a method and apparatus for exercise recommendation, a method and apparatus for sleep recommendation, an electronic device, and a storage medium. The method for exercise recommendation includes: acquiring (S201), by a terminal device, historical sleep data and historical exercise data of a target object; obtaining (S202), according to the historical sleep data and the historical exercise data, a target exercise recommendation result of a target time period for the target object; and outputting (S203) information about the target exercise recommendation result.

FIG. 2

EP 4 560 641 A1

**Description**

**TECHNICAL FIELD**

The present application relates to the field of electronic device technologies, and in particular, to methods and apparatuses for exercise recommendation and sleep recommendation, electronic devices, and storage media.

**BACKGROUND**

With the development of electronic device technologies, the electronic device technologies are increasingly being used to monitor and manage users' health, such as exercise monitoring, sleep monitoring, and sedentary reminders. In medicine, a length of time spent with a high-quality life experience is described as a "healthy lifespan". A process of pursuing the "healthy lifespan" is referred to as "proactive health." In order to achieve the goal of "proactive health", it is necessary to assess a user's state in real time and provide the user with appropriate health guidance through a feedback loop similar to that in a cybernetic system, thereby achieving a preset health goal of the user.

The limitation of the related art is that, although a plurality of categories of data may be acquired by an electronic device from the user, the data is not analyzed and utilized at a deeper level to help the user achieve the proactive health goal.

**SUMMARY**

The present application provides methods and apparatuses for exercise recommendation, methods and apparatuses for sleep recommendation, electronic devices, and storage media.

In a first aspect, the present application provides a method for exercise recommendation, including: acquiring, by a terminal device, historical sleep data and historical exercise data of a target object, the historical sleep data including sleep data of at least one day prior to a target time period, and the historical exercise data including exercise data of at least one day prior to the target time period; obtaining, according to the historical sleep data and the historical exercise data, a target exercise recommendation result of the target time period for the target object, the target exercise recommendation result including at least one of a target recommended exercise time or a target recommended exercise amount; and outputting information about the target exercise recommendation result.

According to the technical solution of the present application, the target exercise recommendation result of the target time period for the target object may be acquired based on the historical sleep data and the historical exercise data of the target object, and the target exercise recommendation result may be recommended to the target object, thereby helping the target object maintain a scientific and regular exercise habit and establishing a foundation for improvement of exercise ability of the target object and achievement of the proactive health goal.

In some implementations, the historical sleep data of the target object may be obtained by sleep monitoring on the target object, and may include all or a portion of sleep data of the target object monitored from a certain time point, such as, for example, sleep data in a specific length of time prior to the target time period, or sleep data obtained prior to the target time period that meets a specific condition. The historical exercise data of the target object may be obtained by motion monitoring on the target object, and may include all or a portion of exercise data of the target object monitored from a certain time point, such as, for example, exercise data in a specific length of time prior to the target time period, or exercise data obtained prior to the target time period that meets a specific condition. The historical sleep data and the historical exercise data may be data monitored in the same time period, or data monitored in different time periods, where the different time periods may partially overlap or may not overlap.

In some implementations, the historical sleep data may include at least one of a sleep start time, a sleep end time, a sleep time duration, sleep heart rate data, sleep staging data, or sleep respiration data.

In some implementations, the historical exercise data may include at least one of an exercise start time, an exercise end time, an exercise time duration, an exercise intensity, an exercise type, exercise heart rate data, an exercise impulse, or exercise energy consumption.

In some implementations, the time period takes a day as a unit of time, such as, for example, a calendar day, 24 hours counted from a certain time point, or the like.

For instance, the time point when this method is performed is referred to as a current day, and the target exercise recommendation result of the target time period for the target object includes a target exercise recommendation result of the following day for the target object, such as, for example, a target exercise recommendation result for the current day (e.g., today), or a target exercise recommendation result for the next day (e.g., tomorrow).

In some implementations, the a candidate exercise time set is determined based at least in part on historical exercise time data included in the historical exercise data, where the candidate exercise time set includes at least one candidate exercise time (i.e., at least one candidate exercise time point or at least one candidate exercise time period); and the target recommended exercise time is determined from the candidate exercise time set based at least in part on the historical sleep data.

The candidate exercise time set may include one or more candidate exercise times (i.e., one or more candidate exercise time points or time periods). The historical exercise data may include historical exercise time data. The one or more candidate exercise times may be determined based on a portion or all of the historical exercise time data included in the historical exercise data. For example, all of the exercise times included in the historical exercise time data may be taken as the candidate exercise times. Alternatively, one or more exercise times among all of the exercise times included in the historical exercise time data that meet a certain condition may be taken as the candidate exercise times. For example, an exercise time with a number of occurrence in the historical exercise time data reaching a certain threshold is taken as the candidate exercise time, and so on. Alternatively, the one or more candidate exercise times are obtained by processing part or all of the historical exercise time data.

In some examples, the historical exercise data is used to determine an exercise habit or preference of the target object, and the historical sleep data is used to determine a sleep habit or preference of the target object. The exercise recommendation for the target object may be performed based on the exercise habit or preference as well as the sleep habit or preference of the target object.

According to the technical solutions of the present application, the target recommended exercise time for the target object may be determined based on at least part of the historical sleep data and at least part of the historical exercise data of the target object, thereby helping the target object maintain a scientific and regular exercise habit based on the exercise and sleep habits or preferences of the target object, and establishing a foundation for improvement of exercise ability of the target object and achievement of the proactive health goal.

In some implementations, a sleep type of the target object may be determined, and the target recommended exercise time may be determined from the candidate exercise time set according to the sleep type of the target object and at least part of the historical sleep data.

In some examples, the sleep type of the target object is determined based at least in part on the historical sleep data of the target object. For example, at least part of the historical sleep data of the target object is processed by using a classification model, to obtain the sleep type of the target object.

In some other examples, the sleep type of the target object is determined according to received user input information.

In some implementations, the sleep type of the target object may include one of sleep early, sleep late, wakeup early, wakeup late, and insomnia.

In some implementations, the historical sleep data includes historical sleep start time data, and the target recommended exercise time may be determined from the at least one candidate exercise time based at least in part on the historical sleep start time data.

In some implementations, an exercise time offset corresponding to the sleep type of the target object may be determined from a plurality of preset exercise time offsets; and the target recommended exercise time may be determined from the candidate exercise time set according to the determined exercise time offset and at least part of the historical sleep start time data included in the historical sleep data.

In some examples, an exercise time requirement is determined according to the exercise time offset corresponding to the sleep type of the target object and at least part of the historical sleep start time data included in the historical sleep data; and the target recommended exercise time is determined from one or more candidate exercise times in the candidate exercise time set that meet the exercise time requirement.

In some other examples, the exercise time requirement may be determined based on historical sleep start time data of the target object of at least one day close to the target time period and the exercise time offset corresponding to the sleep type of the target object.

In some examples, the exercise time requirement may include a latest exercise time. The latest exercise time may include a latest exercise start time, and/or a latest exercise end time.

In some implementations, a plurality of candidate recommended exercise times may be selected from the candidate exercise time set based at least in part on the historical sleep data, prompt information is then output to prompt selection from the plurality of candidate recommended exercise times, and the target recommended exercise time is determined based on received user input information.

In some implementations, the target recommended exercise time may be selected from the candidate exercise time set based on at least part of the historical sleep data and at least part of the historical exercise data.

In some examples, a plurality of candidate recommended exercise times may be selected from the candidate exercise time set based at least in part on the historical sleep data, and the target recommended exercise time is selected from the plurality of candidate recommended exercise times based at least in part on the historical exercise data.

For instance, an exercise frequency of the target object at each of the plurality of candidate recommended exercise times is determined according to at least part of the historical exercise data; and the target recommended exercise time is determined from the plurality of candidate recommended exercise times according to the exercise frequency of the target object at each of the plurality of candidate recommended exercise times.

In some implementations, an initial exercise recommendation result of the target time period is determined based on first historical exercise data in the historical exercise data, and adjustment processing is performed on the initial exercise recommendation result based on at least part of the historical sleep data, to obtain the target exercise recommendation result of the target time period.

In some examples, the exercise recommendation may be made in units of an exercise recommendation cycle. Each exercise recommendation cycle may include a plurality of days or time periods. For instance, a length of time included in the exercise recommendation cycle may be preset, such as, for example, a week, or may be user-specified.

In some examples, the first historical exercise data includes exercise data in at least one historical exercise recommendation cycle prior to a current exercise recommendation cycle to which the target time period belongs. For instance, the first historical exercise data includes exercise data in previous N weeks of the current week, where N≥1.

In some other examples, the first historical exercise data includes exercise data in one or more time periods prior to the target time period. For instance, the first historical exercise data includes exercise data in previous N days of the current day.

In some examples, the at least part of the historical sleep data includes sleep data during at least one day close to the target time period. For instance, the at least part of the historical sleep data includes sleep data during a previous day or previous N days of the target time period, or includes sleep data during one or more days that are passed within the current exercise recommendation cycle.

In some examples, an initial exercise recommendation result of each of a plurality of time periods included in the current exercise recommendation cycle may be determined according to the same historical exercise data. For instance, the initial exercise recommendation result of each of the plurality of time periods included in the current exercise recommendation cycle is determined according to exercise data of at least one historical exercise recommendation cycle prior to the current exercise recommendation cycle.

In some other examples, the initial exercise recommendation results of different time periods included in the current exercise recommendation cycle may be determined according to different historical exercise data. For instance, an initial exercise recommendation result of each time period is determined according to exercise data of at least one time period prior to the each time period.

In some implementations, the determination of the initial exercise recommendation result and the determination of the target exercise recommendation result may be performed at different time points. In some examples, the initial exercise recommendation result of the target time period may be determined at a first time point prior to the current exercise recommendation cycle, and a target exercise recommendation result of each time period within the current exercise recommendation cycle is determined at a second time point when or before that time period arrives. For instance, an initial exercise recommendation result for each day of the current week is determined prior to the current week, and on each day of the current week, a target exercise recommendation result of the current day or the next day is determined.

In some implementations, the initial exercise recommendation result of the target time period is determined based on a health state of the target object at the first time point and the first historical exercise data.

In some examples, an exercise recommendation strategy for the target time period is determined according to a physiological measurement result for the target object; and the initial exercise recommendation result of the target time period is determined based on the exercise recommendation strategy for the target time period and the first historical exercise data.

The physiological measurement result for the target object may be obtained by performing one or more physiological measurements on the target object, such as heart rate measurement, blood pressure measurement, psychological stress measurement, and/or the like. The current health state of the target object may be determined according to the physiological measurement result, and the current exercise recommendation strategy for the target object may be determined based on the current health state of the target object.

In some examples, the initial and/or target exercise recommendation result includes a recommended exercise amount. Correspondingly, the exercise recommendation strategy may include at least one of increasing the exercise amount, maintaining the exercise amount, or decreasing the exercise amount.

In some implementations, a sleep quality assessment result for the target object during at least one day close to the target time period is obtained based on at least part of the historical sleep data; and the adjustment processing is performed on the initial exercise recommendation result of the target time period based on the sleep quality assessment result for the target object during the at least one day close to the target time period, to obtain the target exercise recommendation result of the target time period.

In some examples, the sleep quality assessment result of the at least one day close to the target time period may include a sleep quality assessment result of a previous day or previous N days of the target time period, such as, for example, a sleep quality assessment result of previous two weeks, or a sleep quality assessment result of one or more days that are passed within the current exercise recommendation cycle, e.g., a sleep quality assessment result of at least one day that is passed in the current week.

In some implementations, the adjustment processing is performed on the initial exercise recommendation result according to at least part of the historical sleep data and second historical exercise data in the historical exercise data, to obtain the target exercise recommendation result of the target time period, where the second historical exercise data includes exercise data of at least one day that is within the current exercise recommendation cycle and prior to the target time period.

In some examples, the historical exercise data includes the second historical exercise data, and the second historical exercise data includes exercise data during a previous day or previous N days close to the target time period. In some other examples, the second historical exercise data includes exercise data during one or more days that are passed within the current exercise recommendation cycle.

In some implementations, an exercise assessment result for the target object during the at least one day prior to the target time period is obtained according to the second historical exercise data, and the adjustment processing is performed on the initial exercise recommendation result of the target time period according to the exercise assessment result and the at least part of the historical sleep data, to obtain the target exercise recommendation result of the target time period.

In some examples, the sleep quality assessment result for the target object during at least one day prior to the target time period is obtained according to the at least part of the historical sleep data, the exercise assessment result for the target object during at least one day prior to the target time period is determined according to the second historical exercise data, and the adjustment processing is performed on the initial exercise recommendation result according to the sleep quality assessment result and the exercise assessment result, to obtain the target exercise recommendation result of the target time period.

In some implementations, the initial exercise recommendation result of the target time period is determined, and the adjustment processing is performed on the initial exercise recommendation result according to at least part of the historical sleep data and second historical exercise data in the historical exercise data, to obtain the target exercise recommendation result of the target time period.

The second historical exercise data may include exercise data during the at least one day close to the target time period.

The initial exercise recommendation result of the target time period may be obtained according to the historical exercise data and/or the historical sleep data of the target object, for example, with various possible implementations as described above. Alternatively, the initial exercise recommendation result is obtained according to user input information. Alternatively, the initial exercise recommendation result is obtained according to prior knowledge or expert knowledge. In some examples, the initial exercise recommendation result is obtained according to at least one of the first historical exercise data of the target object, a physiological measurement result for the target object, the user input information, or prior information on exercise physiology (or referred to as expert knowledge on exercise physiology).

In some implementations, the exercise assessment result for the target object is obtained according to the second historical exercise data; and the adjustment processing is performed on the initial exercise recommendation result according to the exercise assessment result and the at least part of the historical sleep data, to obtain the target exercise recommendation result of the target time period.

In some implementations, the exercise assessment result for the target object is obtained according to the second historical exercise data; the sleep quality assessment result for the target object is obtained according to the at least part of the historical sleep data; and the adjustment processing is performed on the initial exercise recommendation result according to the exercise assessment result and the sleep quality assessment result, to obtain the target exercise recommendation result of the target time period.

The exercise assessment result described above may be used to indicate an exercise accomplishment condition of the target object during the at least one day close to the target time period.

In some examples, the exercise assessment result includes at least one of an exercise amount accomplishment frequency or a total exercise amount accomplishment indicator. The total exercise amount accomplishment indicator may be used to indicate whether an expected total exercise amount of the target object during the at least one day close to the target time period is achieved. The exercise amount accomplishment frequency may be used to indicate a frequency that an expected exercise amount is achieved during the at least one day, e.g., a ratio of a number of days in which the expected exercise amount is achieved to a total number of days corresponding to the at least one day. For instance, the exercise amount accomplishment frequency may indicate a proportion of days in which an expected exercise amount is achieved during one or more days that are passed within the current exercise recommendation cycle, and the total exercise amount accomplishment indicator may indicate whether a total exercise amount of one or more days that are passed within the current exercise recommendation cycle reaches a set total exercise amount.

In some implementations, an actual exercise amount of the at least one day close to the target time period may be obtained according to the second historical exercise data. In some examples, the second historical exercise data includes the actual exercise amount during the at least one day close to the target time period. In some examples, the second historical exercise data includes an actual exercise amount during each of the at least one day that is passed within the current exercise recommendation cycle. In some other examples, the actual exercise amount during the at least one day close to the target time period may be obtained by processing at least part of the second historical exercise data.

In some implementations, the exercise assessment result is obtained according to an actual exercise amount during each of the at least one day close to the target time period and recommended exercise information corresponding to the at least one day.

In some examples, the recommended exercise information corresponding to the at least one day includes a recommended exercise amount for each of the at least one day. In some other examples, the recommended exercise information corresponding to the at least one day includes a recommended total exercise amount for the at least one day. In some other examples, the recommended exercise information corresponding to the at least one day includes a recommended total exercise amount for the current exercise recommendation cycle.

In some examples, the actual exercise amount of the target object during each of the at least one day close to the target time period is compared with the recommended exercise amount of the day to determine whether the expected exercise amount of the day for the target object is achieved; and the exercise amount accomplishment frequency of the target object during the at least one day is determined according to whether the expected exercise amount of the target object during each of the at least one day close to the target time period is achieved.

In some other examples, an actual total exercise amount of the target object during the at least one day is determined according to the actual exercise amount of the target object during each of the at least one day close to the target time period, and it is determined whether an expected total exercise amount of the at least one day is achieved according to a recommended total exercise amount corresponding to the at least one day and the actual total exercise amount of the at least one day.

In some implementations, a target recommended exercise amount of the target time period is obtained by comparing an exercise amount accomplishment index included in the exercise assessment result with a first preset threshold and/or comparing a sleep quality index included in the sleep quality assessment result with a second preset threshold.

In some examples, in response to the exercise assessment result indicating that the expected total exercise amount is not achieved and the sleep quality assessment result indicating that a sleep quality is lower than a preset quality threshold, the initial recommended exercise amount included in the initial exercise recommendation result is adjusted to a minimum exercise amount of the target object. In this case, the target recommended exercise amount is the minimum exercise amount of the target object.

In some examples, in response to the exercise assessment result indicating that the expected total exercise amount is not achieved and the sleep quality assessment result indicating that the sleep quality reaches the preset quality threshold, the initial recommended exercise amount included in the initial exercise recommendation result is not adjusted. In this case, the target recommended exercise amount is determined as the initial recommended exercise amount.

In some examples, in response to the exercise assessment result indicating that the expected total exercise amount is achieved and the exercise amount accomplishment frequency reaches a preset frequency threshold, the initial recommended exercise amount included in the initial exercise recommendation result may be adjusted to zero to obtain the target recommended exercise amount. In this case, the target recommended exercise amount is zero. That is, no exercise is recommended for the target time period.

In some examples, in response to the exercise assessment result indicating that the expected total exercise amount is achieved and the exercise amount accomplishment frequency is lower than the preset frequency threshold, the initial recommended exercise amount included in the initial exercise recommendation result may be adjusted to the minimum exercise amount of the target object to obtain the target recommended exercise amount. In this case, the target recommended exercise amount is the minimum exercise amount of the target object.

According to the technical solutions in the present application, the initial recommended exercise amount in the target time period may be determined, and the initial recommended exercise amount in the target time period is dynamically adjusted according to actual exercise and sleep condition during one or more days close to the target time period, so that the target recommended exercise amount is more in line with a current state of the target object and the exercise recommendation is more effective.

In some implementations, the sleep quality assessment result includes a sleep recovery index.

In some implementations, sleep heart rate variability (HRV) data of the target object is obtained according to at least part of the historical sleep data; and the sleep recovery index of the target object is obtained based on the sleep HRV data of the target object.

In some examples, the sleep HRV data of the target object includes at least one of a short-term sleep HRV parameter of the target object, a long-term sleep HRV parameter of the target object, or a sleep HRV parameter of the target object during a previous day.

The short-term sleep HRV parameter may indicate HRV characteristics of the target object during sleep in a relatively short time duration close to the target time period. The relatively short time duration may be, for example, a week prior to the target time period, or a previous exercise recommendation cycle of the current exercise recommendation cycle.

In some examples, the short-term sleep HRV parameter may include a baseline HRV.

The long-term sleep HRV parameter may indicate HRV characteristics of the target object during sleep in a relatively long time duration close to the target time period. The relatively long time duration may be, for example, previous two weeks, a previous month, or a longer time duration prior to the target time period or prior to the current exercise recommendation cycle. In some examples, the long-term sleep HRV parameter may include a normal fluctuation range of HRV.

In some implementations, a sleep recommendation result for the target object may be further determined based at least in part on the historical sleep data of the target object. Specific implementations may be found in any possible implementation of a second aspect.

In the second aspect, the present application provides a method for sleep recommendation, including: acquiring, by a terminal device, historical sleep data of a target object; obtaining, according to at least one of attribute information of the target object or expected sleep information of the target object, a target value of at least one sleep parameter of the target object; obtaining a sleep recommendation result of a target time period for the target object based on the target value of the at least one sleep parameter and the historical sleep data of the target object, where the sleep recommendation result includes at least one of a recommended sleep start time, a recommended wakeup time, or a recommended sleep time duration; and outputting information about the sleep recommendation result.

According to the technical solutions in the present application, the target value of the at least one sleep parameter may be obtained according to at least one of the attribute information of the target object or the expected sleep information of the target object, and the sleep recommendation result of the target time period for the target object is obtained based on the target value of the at least one sleep parameter and the historical sleep data of the target object, so as to help the target object gradually develop a scientific and regular sleep habit.

In some examples, the target time period may be the current day, the next day, or the next week. A duration of the target time period may be pre-set or user-specified.

In some examples, the historical sleep data of the target object may include sleep data of one or more time periods prior to the target time period. The historical sleep data of the target object may be used to indicate recent sleep condition of the target object. The historical sleep data may include sleep data of a previous day of the target time period, sleep data of one or more days that are passed within a current sleep recommendation cycle to which the target time period belongs, or sleep data of a previous sleep recommendation cycle. For example, the historical sleep data may include sleep data during a previous day of the target time period, sleep data of one or more days that are passed in the current week, or sleep data of a previous week.

In some examples, the expected sleep information of the target object includes an expected value of the at least one sleep parameter, i.e., an expected value of one or more sleep parameters, such as at least one of an expected sleep time duration, an expected wakeup time, or an expected sleep start time. The expected sleep information may be obtained by receiving an input instruction from the target object, or may be obtained according to sleep information of a group to which the target object belongs.

In some examples, the attribute information of the target object includes personal profile information of the target object, such as at least one of age, gender, a body mass index (BMI), an occupation, a hobby, or a sleep habit.

In some implementations, a validity of the expected sleep information may be determined before the target value of the at least one sleep parameter is obtained.

In some examples, if it is determined that the expected sleep information is valid, the target value of the at least one sleep parameter may be determined according to the expected sleep information.

In some other examples, if it is determined that the expected sleep information is invalid, the target value of the at least one sleep parameter may be determined according to the attribute information of the target object.

In some implementations, a validity of the expected value of each of the at least one sleep parameter included in the expected sleep information may be determined before the target value of the at least one sleep parameter is obtained. For instance, it is determined whether the expected value of a certain sleep parameter is within a preset range, and if the expected value is within the preset range, it is determined to be valid, while if the expected value is not within the preset range, it is determined to be invalid.

In some other examples, if the expected sleep information includes expected values of a plurality of sleep parameters, and the expected values of some sleep parameters are valid and the expected values of others are invalid, a target value of a sleep parameter whose expected value is invalid may be determined based on an expected value of another sleep parameter which is determined to be valid and/or the attribute information of the target object.

In some implementations, in response to the expected sleep information of the target object including a valid expected value corresponding to a first sleep parameter, a target value of the first sleep parameter is determined according to its corresponding valid expected value. For example, the valid expected value corresponding to the first sleep parameter is determined to be the target value of the first sleep parameter.

In some implementations, in response to the expected sleep information of the target object not including a valid expected value corresponding to a second sleep parameter, a target value of the second sleep parameter is determined according to the attribute information of the target object.

In some examples, a user group or sleep group to which the target object belongs may be determined based on the attribute information of the target object, and then the target value of the at least one sleep parameter is determined based on the user group or sleep group to which the target object belongs.

In some implementations, a sleep adjustment strategy for the target object is determined based on at least part of the historical sleep data of the target object and the target value of the at least one sleep parameter; and the sleep recommendation result of the target time period for the target object is obtained according to the sleep adjustment strategy.

The sleep adjustment strategy may include a progressive adjustment strategy or a direct adjustment strategy.

In some implementations, a current value of the at least one sleep parameter of the target object may be obtained based on at least part of the historical sleep data of the target object, and the sleep recommendation result is obtained according to the current value of the at least one sleep parameter and the target value of the at least one sleep parameter.

In some examples, the current value of the at least one sleep parameter may include at least one of a historical sleep start time, a historical wakeup time, or a historical sleep time duration.

In some examples, at least part of the historical sleep data of the target object is processed to obtain the current value of the at least one sleep parameter of the target object. For instance, the current value of each of the at least one sleep parameter is a statistical average of the values of the sleep parameter within a time interval prior to the target time period.

In some examples, the sleep recommendation result of the target time period may be obtained by comparing the current value of the at least one sleep parameter and the target value of the at least one sleep parameter.

In some implementations, in response to a difference between the current value of the at least one sleep parameter and the target value of the at least one sleep parameter exceeding a preset difference range, the sleep adjustment strategy for the target object is determined as the progressive adjustment strategy.

In some examples, in response to the difference between the current value of the at least one sleep parameter indicated by at least part of the historical sleep data of the target object and the target value of the at least one sleep parameter exceeding the preset difference range, the sleep adjustment strategy is determined as the progressive adjustment strategy.

In some implementations, in the progressive adjustment strategy, the sleep recommendation result of the target time period is obtained based on an adjustment step size and the current value of the at least one sleep parameter.

For instance, the current value of the at least one sleep parameter is adjusted with the adjustment step size to obtain the sleep recommendation result of the target time period, so that a recommended value of each sleep parameter included in the sleep recommendation result approaches the target value of the sleep parameter from the current value of the sleep parameter.

In some examples, the adjustment step size is determined based on the difference between the current value of the at least one sleep parameter and the target value of the at least one sleep parameter.

In some other examples, the adjustment step size is pre-set or determined according to user input information.

In some implementations, in response to the difference between the current value of the at least one sleep parameter and the target value of the at least one sleep parameter being within the preset difference range, the sleep adjustment strategy is determined as the direct adjustment strategy.

In some examples, in the direct adjustment strategy, the sleep recommendation result is determined based on the target value of the at least one sleep parameter.

In some examples, in the direct adjustment strategy, the recommended value of the at least one sleep parameter included in the sleep recommendation result for the target object is determined as the target value of the at least one sleep parameter.

In some implementations, the method further includes: obtaining a supplemental sleep recommendation result of the target time period for the target object according to sleep data during a previous day of the target time period included in the historical sleep data of the target object.

In some examples, the sleep data during the previous day may include regular sleep data and/or supplemental sleep data during the previous day.

In some implementations, it may be determined whether to recommend the target object for supplemental sleep in the target time period based on whether a regular sleep parameter of the target object during the previous day of the target time period is within a preset parameter range.

In some examples, in response to a time duration of regular sleep of the target object during the previous day not reaching a preset sleep time duration, it is determined that a recommended time duration for supplemental sleep included in the supplemental sleep recommendation result of the target time period for the target object is a first time duration greater than zero. In this case, it is recommended to take a supplemental sleep for a certain length of time in the target time period.

The first time duration may be a preset value, or may be determined based at least in part on the historical sleep data of the target object.

In some other examples, in response to the time duration of the regular sleep of the target object during the previous day reaching the preset sleep time duration, it is determined that the recommended time duration for supplemental sleep included in the supplemental sleep recommendation result for the target object is zero. In this case, it is recommended not to take a supplemental sleep in the target time period.

In some implementations, the method further includes: outputting a warning for a time duration of supplemental sleep based at least in part on the historical sleep data of the target object.

In some implementations, the warning for the time duration of supplemental sleep is output in response to the sleep data during the previous day of the target time period indicating that a time duration for supplemental sleep of the target object during the previous day exceeds a recommended time duration range, where the warning for the time duration of supplemental sleep is used for prompting control of the time duration of supplemental sleep in the target time period to be within the recommended time duration range.

With the technical solutions in the present application, the supplemental sleep recommendation result for the target object may be obtained based on at least part of the historical sleep data of the target object, thereby avoiding the target object being lack of energy due to poor regular sleep and also avoiding adverse effects of a relatively long supplemental sleep on the health, and establishing a foundation for achievement of the proactive health goal of the target object.

In a third aspect, the present application provides an apparatus for exercise recommendation, including: an acquisition module configured to acquire, by a terminal device, historical sleep data and historical exercise data of a target object, the historical sleep data including sleep data of at least one day prior to a target time period, and the historical exercise data including exercise data of at least one day prior to the target time period; a processing module configured to obtain, according to the historical sleep data and the historical exercise data, a target exercise recommendation result of the target time period for the target object, the target exercise recommendation result including at least one of a target recommended exercise time or a target recommended exercise amount; and an output module configured to output information about the target exercise recommendation result.

In some implementations, the apparatus for exercise recommendation includes one or more modules or units configured to perform the various processes and/or steps in the first aspect or in any possible implementation of the first aspect.

In a fourth aspect, the present application provides an apparatus for sleep recommendation, including: an acquisition module configured to acquire, by a terminal device, historical sleep data of a target object; a first processing module configured to obtain, according to at least one of attribute information of the target object or expected sleep information of the target object, a target value of at least one sleep parameter of the target object; a second processing module configured to obtain a sleep recommendation result of a target time period for the target object based on the target value of the at least one sleep parameter of the target object and at least part of the historical sleep data of the target object, the sleep recommendation result including at least one of a recommended sleep start time, a recommended wakeup time, or a recommended sleep time duration; and an output module configured to output information about the sleep recommendation result.

In some implementations, the apparatus for sleep recommendation includes one or more modules or units configured to perform various processes and/or steps in the second aspect or in any possible implementation of the second aspect.

In a fifth aspect, the present application provides an electronic device, including: at least one processor; and a memory communicatively coupled to the at least one processor; where the memory stores instructions executable by the at least one processor, and the instructions are executed by the at least one processor to enable the at least one processor to perform the method for exercise recommendation according to the first aspect or any possible implementation of the first aspect, or to enable the at least one processor to perform the method for sleep recommendation according to the second aspect or any possible implementation of the second aspect.

In a sixth aspect, the present application provides a computer-readable storage medium, configured to store instructions, where, when the instructions are executed, the method for exercise recommendation according to the first aspect or any implementation of the first aspect is implemented, or the method for sleep recommendation according to the second aspect or any implementation of the second aspect is implemented.

In a seventh aspect, the present application provides a computer program product, including a computer program, where, when the computer program is executed by a processor, the method for exercise recommendation according to the first aspect or any implementation of the first aspect is implemented, or the method for sleep recommendation according to the second aspect or any implementation of the second aspect is implemented.

It should be understood that the content described in this section is neither intended to identify key or important features of embodiments of the present application, nor intended to limit the scope of the present application. Other features of the present application will be readily understood from the following description.

## BRIEF DESCRIPTION OF DRAWINGS

The accompanying drawings are used for a better understanding of the present application, and do not constitute a limitation on the present application.

FIG. 1 is an exemplary block diagram of a health guidance system according to some embodiments of the present application.
FIG. 2 is a schematic flowchart of a method for exercise recommendation according to some embodiments of the present application.
FIG. 3 is another schematic flowchart of the method for exercise recommendation according to some embodiments of the present application.
FIG. 4 is another schematic flowchart of the method for exercise recommendation according to some embodiments of the present application.
FIG. 5 is a schematic diagram of an exemplary implementation of exercise amount recommendation according to some embodiments of the present application.
FIG. 6 is a schematic diagram of an exemplary implementation of the exercise recommendation according to some embodiments of the present application.
FIG. 7 is a schematic flowchart of a method for sleep recommendation according to some embodiments of the present application.
FIG. 8 is a schematic diagram of an exemplary implementation of the sleep recommendation according to some embodiments of the present application.
FIG. 9 is a schematic diagram of an exemplary implementation of supplemental sleep recommendation according to some embodiments of the present application.
FIG. 10 is a schematic diagram of another exemplary implementation of the sleep recommendation according to some embodiments of the present application.
FIG. 11 is a schematic diagram of an exemplary health guidance system according to some embodiments of the present application.
FIG. 12 is a schematic diagram of an apparatus for exercise recommendation according to some embodiments of the present application.
FIG. 13 is a schematic diagram of an apparatus for sleep recommendation according to some embodiments of the present application.
FIG. 14 is another schematic diagram of the apparatus for sleep recommendation according to some embodiments of the present application.
FIG. 15 is a schematic block diagram of an electronic device according to some embodiments of the present application.

## DETAILED DESCRIPTION

Exemplary embodiments of the present application will be described below in conjunction with the accompanying drawings, in which various details of the embodiments of the present application are included to facilitate understanding and should be considered as merely exemplary. Therefore, those of ordinary skill in the art should recognize that various changes and modifications may be made to the embodiments described herein without departing from the scope and spirit of the present application. Similarly, for clarity and conciseness, descriptions of well-known functions and structures are omitted from the following description.

In the description of the present application, unless otherwise stated, "/" means "or". For example, the term "A/B" may mean either A or B. The term "and/or" herein describes an association relationship of associated objects and indicates that three relationships may exist. For example, "A and/or B" may indicate A alone, both A and B, and B alone. Various numbers such as "first" and "second" in the present application are merely used for differentiation for ease of description, and are not used to limit the scope of the embodiments of the present application or to indicate a sequence.

Terminal devices such as wearable devices, smart phones, and personal digital assistants are increasingly used to monitor users' exercise and physiological information, such as heart rate and blood oxygen level. The detected user data is not analyzed and utilized in a deeper level to help the users achieve the proactive health goal.

The embodiments of the present application solve such problems by providing users with exercise recommendation and sleep recommendation based on sleep data and exercise data collected by a terminal device. Historical sleep data and historical exercise data of a target object are collected by a terminal device, a target exercise recommendation result for the target object is obtained according to the historical sleep data and the historical exercise data, and information about the target exercise recommendation result is output. Therefore, the target exercise recommendation result is determined and recommended for the target object based on the historical sleep data and the historical exercise data of the target object, thereby helping the target object maintain a scientific and regular exercise habit and establishing a foundation for improvement of exercise ability of the target object and achievement of the proactive health goal.

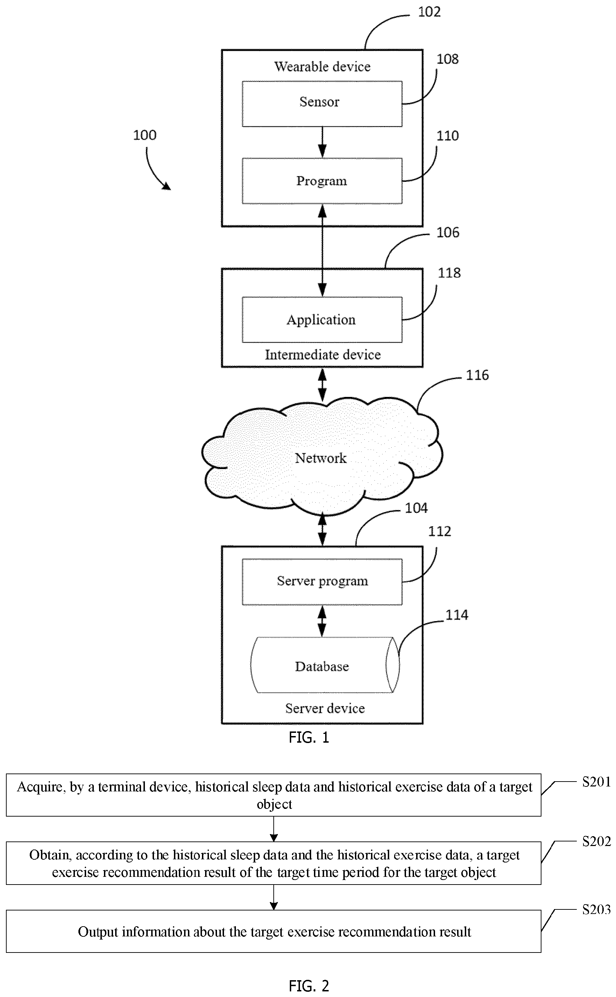

FIG. 1 is an exemplary block diagram of a health guidance system 100 according to some embodiments of the present application. Referring to FIG. 1, the system 100 includes a wearable device 102, a server device 104, and an intermediate device 106. The intermediate device 106 is connected between the wearable device 102 and the server device 104.

The wearable device 102 is a computing device configured to be worn by a user during operation. The wearable device 102 may be implemented as a wrist-worn device worn on the user's wrist, or may be implemented as a band or ring worn on the user's arms, legs, or torso, or may be implemented as a head-mounted device worn on the user's head or eyes, an ear-worn device worn on the user's ears, or a wearable device incorporated in the user's clothing or accessories, or may be implemented as a wearable device worn on the user's waist, legs, or feet, or the like.

Generally, the wearable device 102 may include a housing, a wearing member, a display, a communication unit, a positioning unit, one or more sensors, a memory, and a processor.

In some examples shown in FIG. 1, the wearable device 102 includes one or more sensors 108. The sensors 108 may include at least one of a photoplethysmography (PPG) sensor, a pulse wave sensor, a motion sensor, a blood pressure sensor, a sleep sensor, an electrocardiogram sensor, a body temperature sensor, a stress sensor, an ultrasonic sensor, an infrared sensor, and/or the like. One or more of the sensors 108 may be configured to measure physiological parameters of the user. For example, the physiological parameters may include at least one of heart rate, heart rate variability (HRV), blood oxygen saturation, blood pressure, blood glucose, body temperature, respiration, and/or the like.

In addition, a program 110 runs on the wearable device 102 to process measurement signals generated based on user's data collected by the one or more sensors 108.

The server device 104 is a computing device that runs a server program 112 to process measurement signals or data. The server device 104 may be or may include a hardware server (e.g., a server device), a software server (e.g., a web server and/or a virtual server), or both. For example, in the case that the server device 104 is or includes a hardware server, the server device 104 may be a server device located in a rack.

The server program 112 is configured to use the measurement signals or data to detect one or more of a health state, an exercise state, and a sleep state of the user of the wearable device 102, or any combination thereof. For example, the server program 112 may receive the measurement signals or data from the intermediate device 106, and then use the received measurement signals or data to detect one or more of the health state, the exercise state, and the sleep state of the user of the wearable device 102, or any combination thereof. For example, the server program 112 may use the measurement signals or data to determine a user state or a change in the user state, and then detect at least one of the health state, the exercise state, or the sleep state of the user of the wearable device 102, or any combination thereof based on the determined user state or change in the user state.

The server program 112 may access a database 114 in the server device 104 to perform at least some of functions of the server program 112. The database 114 is a database or other data storages configured to store, manage, or otherwise provide data used for fulfilling the functions of the server program 112. For example, the database 114 may store physiological signals or data received by the server device 104 and information generated from or otherwise determined from the physiological signals or data. For example, the database 114 may be a relational database management system, an object database, an XML database, a configuration management database, a management information base, one or more flat files, other suitable non-transitory storage mechanisms, or any combination thereof.

The intermediate device 106 is a device configured to facilitate communication between the wearable device 102 and the server device 104. In particular, the intermediate device 106 receives data from the wearable device 102, and sends the received data to the server device 104, for use by the server program 112 for example. The intermediate device 106 may be a computing device, such as a mobile device (e.g., a smart phone, a tablet computer, a laptop computer, or other types of mobile devices) or other computers (e.g., a desktop computer or other types of non-mobile computers). Alternatively, the intermediate device 106 may be or may include a network hardware, such as a router, a switch, a load balancer, another network device, or any combination thereof. As another alternative, the intermediate device 106 may be other types of network connection devices. For example, the intermediate device 106 may be a networked power charger for the wearable device 102.

For instance, depending on particular implementations of the intermediate device 106, the intermediate device 106 may run an application program 118. The application program 118 may be one or more application softwares installed on the intermediate device 106. In some implementations, the application software may be installed on the intermediate device 106 by a user of the intermediate device 106 (which is typically the same as the user of the wearable device 102, and may not be the same as the user of the wearable device 102 in certain cases) after the intermediate device 106 is purchased. Alternatively, the application software may be installed on the intermediate device 106 in advance by a manufacturer of the intermediate device 106 before the intermediate device 106 leaves the factory. The application program 118 is configured to send data to the wearable device 102 or receive data from the wearable device 102, and/or, to send data to the server device 104 or receive data from the server device 104. The application program 118 may receive a command from the user of the intermediate device 106. The application program 118 may receive the command from the user through a user interface of the application program 118. For example, in the case that the intermediate device 106 is a computing device with a touch screen display, the user of the intermediate device 106 may issue the command by touching at least a portion of the display that corresponds to a user interface element in the application program.

For example, the command received by the application program 118 from the user of the intermediate device 106 may be a command for transmitting the physiological signals or data received at the intermediate device 106 (e.g., from the wearable device 102) to the server device 104. The intermediate device 106 sends the physiological signals or data to the server device 104 in response to the command. In some other examples, the command received by the application program 118 from the user of the intermediate device 106 may be a command for checking information received from the server device 104, such as, for instance, information about one or more of the detected health status, exercise status, and sleep status of the user of the wearable device 102, or any combination thereof.

In some implementations, a client device is given access to the server program 112. In some examples, the client device may be a mobile device, such as a smart phone, a tablet computer, or a laptop computer. In some other examples, the client device may be a desktop computer or other types of non-mobile computers. The client device may run a client application to communicate with the server program 112. For instance, the client application may be a mobile application capable of accessing some or all of the functions and/or data of the server program 112. For instance, the client device may communicate with the server device 104 over a network 116. In some of such implementations, the client device may be the intermediate device 106.

In some implementations, the server device 104 may be a virtual server. For example, the virtual server may be implemented with a virtual machine (e.g., a Java virtual machine). The virtual machine may be implemented with one or more virtual software systems, such as HTTP servers, java servlet containers, hypervisors, or other software systems. In some of such implementations, the one or more virtual software systems used to implement the virtual server may instead be implemented in hardware.

In some implementations, the intermediate device 106 receives data from the wearable device 102 by using a short-range communication protocol. For example, the short-range communication protocol may be Bluetooth®, Bluetooth® Low Energy, Infrared, Z-Wave, ZigBee, or other types of protocols, or any combination thereof. The intermediate device 106 sends the data received from the wearable device 102 to the server device 104 over the network 116. For example, the network 116 may be a local area network, a wide area network, a machine-to-machine network, a virtual private network, or other types of public or private networks. The network 116 may use a remote communication protocol. For example, the remote communication protocol may be Ethernet, TCP, IP, Powerline Communications, Wi-Fi, GPRS, GSM, CDMA, other types of protocols, or any combination thereof.

The system 100 is configured to continuously transmit the physiological signals or data from the wearable device 102 to the server device 104. The one or more sensors 108 may continuously or otherwise frequently and periodically collect the measurement signals or data from the user of the wearable device 102.

Implementations of the system 100 may differ from those shown and described with respect to FIG. 1. In some implementations, the intermediate device 106 may be omitted. In some examples, the wearable device 102 may be configured to communicate directly with the server device 104 over the network 116. For instance, a direct communication between the wearable device 102 and the server device 104 over the network 116 may include the use of a remote low-power system or other types of communication mechanisms. In some other implementations, both the intermediate device 106 and the server device 104 may be omitted. For example, the wearable device 102 may be configured to perform the above-mentioned functions of the server device 104. At Such implementations, the wearable device 102 may process and store data on its own without needing other computing devices.

The method provided by the embodiments of the present application may be performed by an electronic device. Specifically, the method may be performed by a wearable device, or may be performed by another device. In some examples, the wearable device may send the collected data to the intermediate device, and the intermediate device performs the methods. In some other examples, the wearable device may directly send the collected data to the server device, and the server device performs the methods. In some other examples, the wearable device may send the collected data to the intermediate device, the intermediate device sends the data to the server device, and the server device performs the methods. Alternatively, the method according to the embodiments of the present application may be completed jointly by a plurality of devices. In some examples, some operations or steps are performed by the wearable device, and the other operations or steps are performed by the intermediate device. In some other examples, some operations or steps are performed by the wearable device, and the other operations or steps are performed by the server device. In some other examples, some operations or steps are performed by the intermediate device, and the other operations or steps are performed by the server device, which is not limited in the embodiments of the present application.

In some embodiments, the methods may be implemented by an electronic device such as a smart phone, a personal digital assistant, or a tablet computer. For instance, the smart phone collects exercise and/or sleep data from the user, and performs the method according to the embodiments of the present disclosure.

Referring to FIG. 2, FIG. 2 is a schematic flowchart of the method for exercise recommendation according to some embodiments of the present application. The method for exercise recommendation may include, but is not limited to, S201 to S203.

At S201, historical sleep data and historical exercise data of a target object are acquired by a terminal device.

In particular, the historical sleep data includes sleep data during at least one day prior to a target time period, and the historical exercise data includes exercise data during the at least one day prior to the target time period. In some examples, the historical sleep data includes sleep data of a first time period, and the historical exercise data includes exercise data of a second time period.

The time period in various embodiments of the present application may be in units of days, and different time periods may have same or different lengths of time. For example, a time period may include one day, three days, one week, three weeks, one month, six months, or the like. The term "day" herein may refer to a calendar day, or a 24-hour period starting from a certain time point.

In the embodiments of the present application, the time point when the method is performed is referred to as a current day for convenience of description. The first time period may include one or more days prior to the current day, and the second time period may include one or more days prior to the current day. In some embodiments, the first time period may be the same as the second time period. For example, each of the first time period and the second time period includes one or more days prior to the current day. In some examples, each of the first time period and the second time period includes one or more days that are passed in the current week, a previous day of the current day, previous 7 days of the current day, previous 14 days of the current day, a previous month of the current day, previous three months of the current day, etc. In some other examples, each of the first time period and the second time period includes a previous week of a current week to which the current day belongs, previous two weeks of the current week, a previous month of the current week, previous six months of the current week, etc. In some other embodiments, the second time period has an inclusion relationship with the first time period. In some examples, a length of time included in the second time period is greater than or equal to that included in the first time period, that is, the second time period includes the first time period. For instance, the second time period includes a plurality of days prior to the current day, and the first time period includes one day prior to the current day. In some other examples, the first time period includes the second time period. In some other embodiments, there is no intersection between the first time period and the second time period. In some examples, the first time period and the second time period have a chronological relationship. For instance, the second time period is prior to the first time period, e.g., the second time period includes one or several weeks prior to the current week, and the first time period includes one or more days prior to the current day within the current week, that is, the first time period includes one or more days that are passed in the current week. In some other examples, there is an intersection between the first time period and the second time period, that is, the first time period is partially overlapped with the second time period.

In some embodiments, the target time period includes a following day, such as, for example, the current day or the next day. In some other embodiments, the target time period includes one or more following weeks, or a time period of a specific length. For ease of understanding, the following description takes the target time period being a day as an example, but the embodiments of the present application are not limited thereto.

In some embodiments, the electronic device performing the method may be a terminal device, such as, for example, the wearable device 102 as shown in FIG. 1. In this case, the terminal device may collect sleep data and/or exercise data of the target object, and store the collected data in a local or remote memory either directly or after one or more processing is performed on the collected data. Then, at S201, the historical sleep data and/or the historical exercise data stored in the local or remote memory are acquired. In some other embodiments, the electronic device performing the method is a device other than the terminal device, such as, for example, the intermediate device 106 or the server device 104 as shown in FIG. 1. In this case, the terminal device may collect sleep data and/or exercise data of the target object, and send the collected data to the electronic device either directly or after one or more processing is performed on the collected data. Correspondingly, at S201, the electronic device receives the historical sleep data and/or the historical exercise data from the terminal device, or the electronic device acquires the historical sleep data and/or the historical exercise data stored in the local or remote memory, or the electronic device performs one or more processing on the received data from the terminal device to obtain the historical sleep data and/or the historical exercise data.

The historical sleep data of the target object may include one or more types of sleep related data. In some embodiments, the historical sleep data of the target object may include sleep-related physiological data collected by one or more sensors, such as one of body movement data, respiration data, and heart rate data of the target object collected during sleep, or any combination thereof. In some other embodiments, the historical sleep data may be obtained based at least in part on the sleep-related physiological data collected by the one or more sensors. For instance, one or more processing, such as feature extraction, may be performed on the sleep-related physiological data to obtain the historical sleep data of the target object. In some examples, the historical sleep data includes one or any combination of sleep start time, sleep latency, sleep time duration, wake-up time, deep sleep duration, deep sleep ratio, sleep staging data, number of awakenings, cumulative awakening time duration, sleep start time regularity, sleep time duration regularity, and/or the like.

The historical exercise data of the target object may include one or more types of exercise-related data. In some embodiments, the historical exercise data of the target object includes motion data and/or exercise-related physiological data collected during exercise. The motion data may be collected by an accelerometer, a gyroscope, and/or other types of motion sensors. The exercise-related physiological data may include one or any combination of heart rate, respiration rate, skin sweat data, and/or the like. In some other embodiments, the historical exercise data may be obtained based on the collected motion data and/or exercise-related physiological data. For instance, one or more processing may be performed on the collected motion data and/or exercise-related physiological data to obtain the historical exercise data of the target object. In some examples, the historical exercise data includes one or any combination of an exercise type, an exercise start time, an exercise end time, an exercise duration, an exercise impulse, an exercise energy consumption, an exercise intensity, and/or the like.

At S202, a target exercise recommendation result of the target time period for the target object is obtained according to the historical sleep data and the historical exercise data.

In the embodiments of the present application, the target exercise recommendation result includes at least one of a target recommended exercise time or a target recommended exercise amount. The target recommended exercise time may include at least one of an exercise start time or an exercise end time. The target recommended exercise amount may include at least one of a target exercise impulse, a target exercise intensity, an exercise duration at a specific exercise intensity, a target exercise duration, or a target energy consumption. Alternatively, the target recommended exercise time and the target recommended exercise amount may include other information, which is not limited in the embodiments of the present application. In some embodiments, the target exercise recommendation result may further include an exercise type and/or other exercise parameters.

In some embodiments, a sleep type of the target object is determined, and the target recommended exercise time of the target time period is obtained according to the sleep type, at least part of the historical exercise data, and at least part of the historical sleep data of the target object. The sleep type of the target object may be obtained by analyzing user personal information, or, the sleep type of the target object is determined according to user input information. For example, the target object inputs information about his/her own sleep type. Alternatively, the sleep type of the target object is obtained based at least in part on the historical sleep data of the target object. For example, the historical sleep data of the target object in a certain time interval is input into a machine learning model for processing, to obtain the sleep type of the target object.

In some examples, the sleep type of the target object may be determined based at least in part on the historical sleep data of the target object, at least one historical exercise time of the target object is determined according to at least part of the historical exercise data of the target object, and the target recommended exercise time in the target time period is obtained according to the sleep type of the target object and the at least one historical exercise time of the target object.

In some other examples, the sleep type of the target object may be determined based at least in part on the historical sleep data of the target object, at least one candidate exercise time of the target object is determined according to at least part of the historical exercise data of the target object, and the target recommended exercise time is determined from the at least one candidate exercise time based at least in part on the sleep type.

In some embodiments, an initial exercise recommendation result in the target time period is determined for the target object according to at least part of the historical exercise data of the target object, and the adjustment processing is performed on the initial exercise recommendation result based at least in part on the historical sleep data of the target object, to obtain the target exercise recommendation result of the target time period.

In some examples, the initial exercise recommendation result of the target time period is determined according to first historical exercise data in the historical exercise data, and adjustment processing is performed on the initial exercise recommendation result based on at least part of the historical sleep data, to obtain the target exercise recommendation result.

The first historical exercise data includes exercise data of at least one time period prior to the target time period. In some embodiments, the exercise recommendation may be made for an exercise recommendation cycle including a plurality of time periods. In this case, the first historical exercise data may include exercise data of at least one time period prior to a current exercise recommendation cycle to which the target time period belongs. For example, the first historical exercise data may include exercise data of at least one historical exercise recommendation cycle prior to the current exercise recommendation cycle. In some examples, the exercise recommendation cycle is a week, and the first historical exercise data includes exercise data in one or more weeks close to the current week, such as a previous week, previous three weeks, a previous month, or the like.

The at least part of the historical sleep data may include sleep data during one or more days close to the target time period. For example, the at least part of the historical sleep data may include sleep data of a previous day or previous $N$ days of the target time period. For another example, the at least part of the historical sleep data may include sleep data of one or more days within the current exercise recommendation cycle. For yet another example, the at least part of the historical sleep data may include sleep data during one or more days that are passed within the current exercise recommendation cycle.

In some examples, based on the first historical exercise data, an initial exercise recommendation result (e.g., an initial recommended exercise time and/or an initial recommended exercise amount) is determined for each of a plurality of time periods included in the current exercise recommendation cycle to which the target time period belongs. In this case, the initial exercise recommendation result for each of the plurality of time periods may be obtained by combining expert knowledge or exercise physiology knowledge or exercise strategy information determined according to the exercise physiology knowledge with the first historical exercise data of the target object.

The determination of the initial exercise recommendation result and the determination of the target exercise recommendation result may be performed at different time points. In some examples, the initial exercise recommendation result for each day of the current week may be determined prior to the current week, while on each day of the current week, the target exercise recommendation result for the current day or the next day may be determined. In this case, the initial exercise recommendation result of the target time period is determined at a first time point according to the first historical exercise data. The first time point is prior to the current exercise recommendation cycle to which the target time period belongs. The adjustment processing is performed on the initial exercise recommendation result at a second time point according to the at least part of the historical sleep data, to obtain the target exercise recommendation result of the target time period. The second time point is within the current exercise recommendation cycle and prior to the target time period. For example, the second time point is a previous day or the current day of the target time period.

In some embodiments, a sleep quality assessment result for the target object during at least one day close to the target time period is obtained according to the at least part of the historical sleep data, and the adjustment processing is performed on the initial exercise recommendation result based at least in part on the sleep quality assessment result, to obtain the target exercise recommendation result of the target time period.

The sleep quality assessment result may be used for evaluating the user's sleep condition. For example, the sleep quality assessment result may include one or more sleep assessment indexes such as a sleep recovery index, a sleep quality score, and/or a total deep-sleep time duration.

In some examples, the adjustment processing may be performed on the initial recommended exercise amount in the initial exercise recommendation result according to the sleep quality assessment result. For instance, if the sleep quality assessment result indicates that recent or current sleep quality of the target object is relatively poor, e.g., the sleep assessment index is lower than a preset threshold, the initial recommended exercise amount may be decreased to obtain the target recommended exercise amount. For another instance, if the sleep quality assessment result indicates that the recent or current sleep quality of the target object is relatively good, e.g., the sleep assessment index reaches the preset threshold, the initial recommended exercise amount may be increased or maintained to obtain the target recommended exercise amount.

In some other embodiments, the adjustment processing may be performed on the initial exercise recommendation result of the target time period according to at least one of the second historical exercise data included in the historical exercise data or at least part of the historical sleep data, to obtain the target exercise recommendation result of the target time period.

The second historical exercise data may include exercise data during one or more days prior to the target time period. The second historical exercise data may be part of the first historical exercise data. Alternatively, the second historical exercise data may include exercise data during one or more days close to the target time period. In some examples, the second historical exercise data includes exercise data during one or more days within the current exercise recommendation cycle, such as, for example, exercise data during one or more days that are passed within the current exercise recommendation cycle.

In some examples, an exercise assessment result for the target object is obtained according to the second historical exercise data, where the exercise assessment result indicates an exercise accomplishment condition during the at least one day close to the target time period, and the adjustment processing is performed on the initial exercise recommendation result based at least in part on the exercise assessment result for the target object, to obtain the target exercise recommendation result.

In some examples, the exercise accomplishment condition may include an exercise amount accomplishment frequency and/or a total exercise amount accomplishment indicator. The total exercise amount accomplishment indicator may be used for indicating whether an expected total exercise amount of the at least one day close to the target time period is achieved. The exercise amount accomplishment frequency may be used for indicating a frequency of which an expected exercise amount is achieved during the at least one day, e.g., a ratio of the number of days in which the expected exercise amount is achieved to a total number of days corresponding to the at least one day. For example, the exercise amount accomplishment frequency may indicate a proportion of the number of days in which an expected exercise amount is achieved during one or more days that are passed within the current exercise recommendation cycle, and the total exercise amount accomplishment indicator may indicate whether a total exercise amount during one or more days that are passed within the current exercise recommendation cycle reaches a set total exercise amount. The set total exercise amount may be a sum of recommended exercise amounts of the at least one day that are passed, or a recommended total exercise amount of the current exercise recommendation cycle. In some other examples, the exercise accomplishment condition may be characterized by other indexes, which is not limited in the embodiments of the present application.

In some examples, if an exercise amount accomplishment index included in the exercise accomplishment condition is relatively low, e.g., the exercise amount accomplishment index is lower than a preset threshold, the initial recommended exercise amount may be kept unchanged, or the initial recommended exercise amount may be increased. In some other examples, if an exercise amount accomplishment index included in the exercise accomplishment condition is relatively high, e.g., the exercise amount accomplishment index reaches a preset threshold, the initial recommended exercise amount may be kept unchanged, or the initial recommended exercise amount may be decreased.

In some other embodiments, the initial exercise recommendation result of the target time period may be determined, and the adjustment processing is performed on the initial exercise recommendation result of the target time period according to at least part of the historical sleep data and the second historical exercise data in the historical exercise data, to obtain the target exercise recommendation result of the target time period.

The at least part of the historical sleep data includes sleep data during the at least one day close to the target time period, such as, for example, sleep data during a previous day or previous N days of the target time period, or sleep data in one or more time periods with a time interval starting from the target time period less than a set value, or sleep data in one or more time periods between a set time point and the target time period, or sleep data during one or more days that are passed within the current exercise recommendation cycle. The second historical exercise data includes exercise data of the at least one day close to the target time period, such as, for example, exercise data of a previous day or previous N days of the target time period, or exercise data of one or more time periods with a time interval from the target time period less than a set value, or exercise data in one or more time periods between a set time point and the target time period, or exercise data of one or more days that are passed within the current exercise recommendation cycle. In some examples, the second historical exercise data and the at least part of the historical sleep data correspond to the same time period, such as, for example, a time period that is within the current exercise recommendation cycle and prior to the target time period.

In some implementations, the historical sleep data of the target object may be quantitatively assessed to obtain a sleep quality assessment result for the target object. An exercise assessment result may be obtained based on the second historical exercise data, and the adjustment processing may be performed on the initial exercise recommendation result based on the sleep quality assessment result and the exercise assessment result, to obtain the target exercise recommendation result.

At S203, information about the target exercise recommendation result is output.

In some embodiments, the electronic device may push the information about the target exercise recommendation result in any appropriate manner. For example, the electronic device may display, on a screen, the information about the target exercise recommendation result, or output the information about the target exercise recommendation result to the target object by means of notification, voice, vibration or other haptic output.

In some other embodiments, the electronic device may send the information about the target exercise recommendation result to a terminal device, so that the terminal device outputs the information about the target exercise recommendation result to the target object.

The information about the target exercise recommendation result may include the target exercise recommendation result itself, such as, for example, at least one of the target recommended exercise time or the target recommended exercise amount. Alternatively, the information about the target exercise recommendation result may include reminder information generated according to the target exercise recommendation result, such as a reminder for reminding to do exercise at the target recommended exercise time, a reminder generated before doing exercise for reminding to accomplish the target recommended exercise amount, or a reminder generated during exercise for reminding a specific amount of exercise remaining to be done, which is not limited in the embodiments of the present application.

In the embodiments of the present application, the target exercise recommendation result for the target object is obtained based on the historical sleep data and the historical exercise data of the target object, and the information about the target exercise recommendation result is output, thereby facilitating the target object to maintain a scientific and regular exercise habit, and establishing a foundation for improvement of exercise ability of the target object and achievement of the proactive health goal.

The following will describe how to obtain the sleep quality assessment result for the target object according to at least part of the historical sleep data in conjunction with specific examples.

In some examples, the historical sleep data includes at least one of the following sleep parameters: sleep start time, sleep time duration, wake-up time, deep sleep duration, deep sleep ratio, sleep staging, number of awakenings, cumulative awakening time duration, sleep start time regularity, or sleep time duration regularity.

The sleep quality assessment result for the target object may be determined based on one or more of the sleep parameters included in the historical sleep data. In some examples, feature extraction processing may be performed on sleep related physiological data included in the historical sleep data to obtain sleep feature data, and the sleep quality assessment result may be obtained based on the sleep feature data. In some other examples, at least part of the historical sleep data or at least part of the sleep feature data obtained according to the historical sleep data may be pre-processed to obtain input data, and then the input data may be input to a pre-trained sleep quality assessment model to obtain the sleep quality assessment result for the target object.

In some examples, taking the pre-processing of the sleep feature data as an example, the sleep feature data of one or more items may be pre-processed by using a z-score algorithm, which is represented by the following formula for example:

$$z_i = \frac{x_i - u}{s}$$

**where** $z_i$ denotes a standard score of the $i^{th}$ feature in the sleep feature data of a certain item, $x = \{x_1, x_2 ... x_n\}$ denotes a sleep feature set, $n$ denotes the number of features included in the sleep feature set, $x_i$ denotes the $i^{th}$ feature in the sleep feature set, $i$ is an integer, and $1 \leq i \leq n$, $u$ denotes an average value of the sleep feature data, and s denotes a standard deviation of the sleep feature data.

In some examples, the sleep quality assessment may be performed by using a Pittsburgh sleep quality assessment model. The sleep quality assessment model may be implemented by a deep learning network. For instance, the sleep quality assessment model may be expressed as follows:

$$PSQI = DNN(TST, ST, DR, STR, TSTR, WN, WT)$$

where *PSQI* (Pittsburgh Sleep Quality Index) denotes output of the sleep quality assessment model, *DNN* denotes the deep neural network, and *TST, ST, DR, STR, TSTR, WN, WT* denote input of the sleep quality assessment model, where *TST* denotes the total sleep time duration, *ST* denotes the sleep start time, *DR* denotes the deep sleep ratio, *STR* denotes the sleep start time regularity, *TSTR* denotes the total sleep time duration regularity, *WN* denotes the number of awakenings, and *WT* denotes the cumulative awakening time duration.

In some embodiments, the plurality of sleep parameters or the sleep feature data mentioned above may have a same weight for the quality index. In some other embodiments, weights of the above-mentioned sleep parameters or the sleep feature data may be set respectively based on a *PSQI* questionnaire in the sleep medicine field. The above-mentioned deep neural network may be trained in any appropriate manner, e.g., in a supervised or unsupervised manner, which is not limited in the embodiments of the present application.

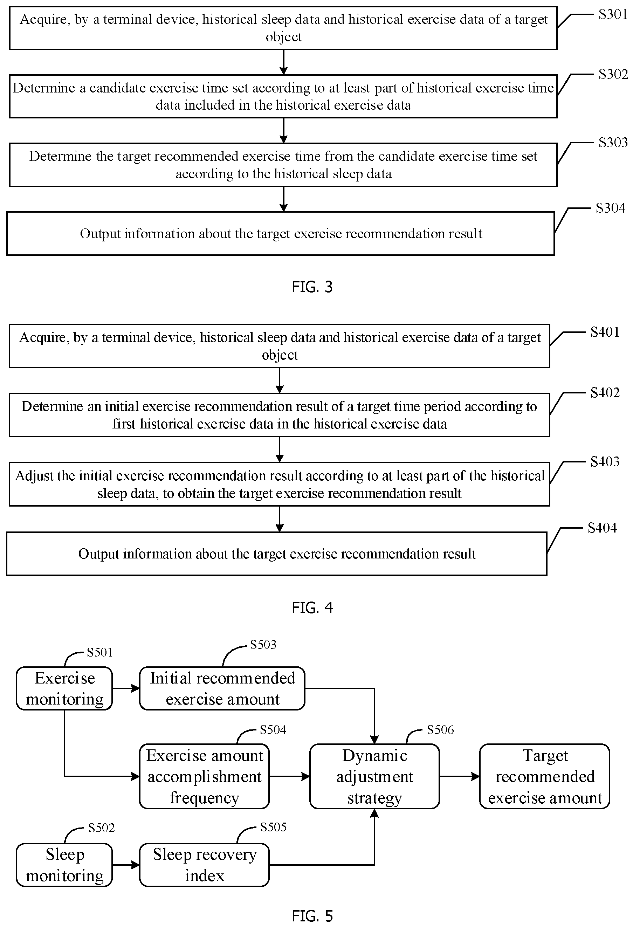

Referring to FIG. 3, FIG. 3 is another schematic flowchart of the method for exercise recommendation according to some embodiments of the present application. In this example, the target exercise recommendation result includes the target recommended exercise time. As shown in FIG. 3, the method may include, but is not limited to, S301 to S304.

At S301, historical sleep data and historical exercise data of a target object are acquired by a terminal device.

S301 may be implemented in any manner described in various embodiments of the present application, and will not be repeated herein.

At S302, a candidate exercise time set is determined based at least in part on historical exercise time data included in the historical exercise data.

In some examples, the historical sleep data includes sleep data of a previous day or time period of the target time period. The historical exercise data includes exercise data of a plurality of days or time periods prior to the target time period, such as one month or three months.

In some examples, the historical exercise data is used for determining an exercise habit or preference of the target object, and the historical sleep data is used for determining a sleep habit or preference of the target object. The recommendation of exercise time for the target object may be made based on the exercise habit or preference of the target object as well as the sleep habit or preference of the target object.

In some examples, the candidate exercise time set may include a plurality of candidate exercise times (e.g., time points or time periods). For instance, all of the historical exercise times included in the historical exercise time data may be taken as the candidate exercise time. Alternatively, among all of the historical exercise times included in the historical exercise time data, one or more exercise times that meet a certain condition may be taken as the candidate exercise time. For instance, a historical exercise time point or period in the historical exercise time data whose number of occurrence reaches a certain threshold is taken as the candidate exercise time, etc. Alternatively, at least a part of the plurality of candidate exercise times is obtained by processing a plurality of historical exercise times in the historical exercise time data. For instance, the plurality of candidate exercise times are obtained by performing statistical analysis on a plurality of historical exercise times included in the historical exercise time data.

In some examples, a plurality of time intervals, to which some or all of the historical exercise times in the historical exercise data belong, are determined, and the determined plurality of time intervals form the candidate exercise time set, *ExtHistory,* which may be expressed as follows:

$$ExtHistory = \{Ext\_1, Ext\_2, \ldots Ext\_n\}$$

where $Ext\_1$, $Ext\_2$, ... $Ext\_n$ denote the time intervals (e.g., with a length of one hour) to which the historical exercise times included in the historical exercise data of the target object belong, n is a positive integer, and $0 \leq n \leq 24$.

At S303, the target recommended exercise time is determined from the candidate exercise time set based at least in part on the historical sleep data.

In some examples, the target recommended exercise time suitable for the sleep condition of the target object is determined from the candidate exercise time set based on at least part of the historical sleep data of the target object and prior knowledge of sleep.

In some implementations, the sleep type of the target object may be determined, and the target recommended exercise time may be determined from the candidate exercise time set according to the sleep type of the target object.

The sleep type of the target object is used for characterizing a sleep habit or pattern of the target object. In some examples, the sleep type may include wakeup early, wakeup late, or insomnia. The type of wakeup early indicates that the target object has a sleep pattern of getting up early. The type of wakeup late indicates that the target object has a sleep pattern of getting up late. The type of insomnia indicates that the target object has a suspected symptom of sleeplessness. In some other examples, the sleep type may include other types, such as one or any combination of sleep early, sleep late, having trouble in falling asleep, dreaming frequently, waking up frequently in the middle of night, having little sleep, or sleeping a lot, which is not limited in the embodiments of the present application.

In some examples, the sleep type of the target object may be obtained according to user input information or an instruction input by the target object. For instance, the target object is prompted by reminder information to input information about his/her sleep habit, such as information about sleep time, whether he/she suffers from insomnia, the sleep type, or the like, and then the sleep type of the target object may be obtained according to received user input information. For instance, the user input information includes sleep type information, and a sleep type corresponding to the sleep type information is taken as the sleep type of the target object. For another instance, the user input information is processed, e.g., the user input information is taken as an input parameter of a sleep classification model to obtain the sleep type of the target object, or the user input information and a preset mapping rule are used to determine the sleep type of the target object.

In some other examples, the sleep type of the target object is obtained by performing sleep monitoring on the target object. For instance, the sleep type of the target object is obtained according to historical sleep data of the target object over a period of time, e.g., the historical sleep data of the target object over the period of time is input, either directly or after one or more processing is performed on the historical sleep data, into a sleep classification model for processing, to obtain the sleep type of the target object. The period of time may be a relatively long period of time. For instance, the period of time may be one month, three months, sixth months, or the like, which is not limited herein. In some implementations, sleep monitoring of the target object may be performed with a wearable device worn by the target object, or the sleep monitoring of the target object may be performed by using other types of terminal devices. In this case, the historical sleep data may be all of the monitored sleep data of the target object obtained by the wearable device or other types of terminal devices, or, the historical sleep data may be part of the monitored sleep data of the target object obtained by the wearable device or other types of terminal devices, such as sleep data satisfying a specific condition or sleep data within a certain time interval from the target time period, which is not limited herein.

In some examples, one or more types of data of the target object, such as sleep latency, sleep start time, sleep end time, number of awakenings, cumulative awakening time duration, deep sleep duration, light sleep duration, and/or the like, are determined based at least in part on the historical sleep data of the target object, and the sleep type of the target object is obtained by performing statistical analysis on the obtained one or more types of data. For instance, if the sleep end time of the target object is usually earlier than or at a preset time point (e.g., 7:00 a.m.), the sleep type of the target object is determined as the wakeup early. For another instance, if the sleep end time of the target object is usually later than a preset time point (e.g., 9:00 a.m.), the sleep type of the target object is determined as the wakeup late. For another instance, if the sleep latency of the target object is greater than a preset time duration, or the number of awakenings during sleep is greater than a preset number of times, or the cumulative awakening time duration during sleep is greater than a preset time duration threshold (e.g., 1 hour), the sleep type of the target object is determined as the insomnia.

In some other examples, the sleep type of the target object is determined in other manners. For instance, the sleep type of the target object is determined according to user profile information, such as personal information of the target object, which is not limited in the embodiments of the present application.

In some implementations, according to the sleep type of the target object, an exercise time that matches the sleep type of the target object may be selected from the candidate exercise time set and taken as the target recommended exercise time. In some examples, an exercise time offset corresponding to the sleep type of the target object is determined from a plurality of preset exercise time offsets, and the target recommended exercise time is determined from the candidate exercise time set according to the determined exercise time offset. For instance, a mapping relationship between sleep types and the preset exercise time offsets may be set in advance, and the exercise time offset corresponding to the sleep type of the target object may be determined by means of search and comparison. For another instance, an exercise time offset determination model may be established in advance, and the sleep type of the target object is processed by using the exercise time offset determination model established in advance, to obtain the corresponding exercise time offset. Alternatively, the corresponding exercise time offset may be determined in other manners, which is not limited in the embodiments of the present application.

In some implementations, the target recommended exercise time is determined from the candidate exercise time set according to the sleep type and at least part of the historical sleep data of the target object.

In some implementations, a reference sleep start time of the target object is determined based on at least part of the historical sleep data and/or the sleep type of the target object; and the target recommended exercise time is selected from the candidate exercise time set according to the corresponding exercise time offset and the reference sleep start time.

In some examples, the reference sleep start time of the target object is determined according to historical sleep start time data included in the historical sleep data. For instance, a sleep start time of the target object on a previous day is taken as the reference sleep start time of the target object. For another instance, the reference sleep start time of the target object is obtained by performing statistical analysis on the historical sleep start times of the target object in a previous week or the first few days of the current week.

In some other examples, a group sleep start time corresponding to the sleep type of the target object is taken as the reference sleep start time of the target object. The group sleep start time may be obtained by analyzing sleep data of a user group corresponding to the sleep type of the target object.

In some implementations, an exercise time requirement is determined according to the exercise time offset and the reference sleep start time of the target object, and the target recommended exercise time is determined from the candidate exercise time set according to the exercise time requirement.

In some examples, the exercise time requirement is determined according to the historical sleep start time data included in the historical sleep data and the exercise time offset; and at least one candidate exercise time in the candidate exercise time set that meets the exercise time requirement is determined as the target recommended exercise time.

In some examples, a required latest exercise time may be determined for the target object according to the exercise time offset and the reference sleep start time, and then one or more candidate exercise times (e.g., time points or periods) in the candidate exercise time set that are prior to the required latest exercise time are taken as the target recommended exercise time.

For instance, the exercise time requirement for the target object is determined according to the following formula:

$$ExtSleepRule = \begin{cases} \{Ext \mid Ext \leq ST_{t-1} - C2\} \; if \; Wakeup \; Early \\ \{Ext \mid Ext \leq ST_{t-1} - C3\} \; if \; Wakeup \; Late \\ \{Ext \mid Ext \geq ST_{t-1} - C4\} \quad if \; Insomnia \end{cases}$$

where *ExtSleepRule* denotes a candidate time meeting the exercise time requirement, $ST_{t-1}$ denotes a sleep start time in a previous time period or a previous exercise recommendation cycle, e.g., a sleep start time of the target object on a previous day or in a previous week, and *C2*, *C3*, and *C4* denote exercise time offsets corresponding to the type of wakeup early, wakeup late, and insomnia, respectively. An intersection between the time intervals in the candidate exercise time set and the time intervals meeting the exercise time requirement is determined to obtain a candidate recommended exercise time, which may be expressed as follows as an example:

$$ExtCandidate = ExtHistory \cap ExtSleepRule$$

where *ExtCandidate* denotes the candidate recommended exercise time set including one or more candidate recommended exercise times selected from the candidate exercise time set, *ExtHistory* denotes the candidate exercise time set, and *ExtSleepRule* denotes a set of exercise times meeting the exercise time requirement. At least one candidate recommended exercise time in the candidate recommended exercise time set may be determined as the target recommended exercise time.

In some implementations, the candidate recommended exercise time set includes only one candidate recommended exercise time. In this case, the candidate recommended exercise time may be determined as the target recommended exercise time. In some other implementations, the candidate recommended exercise time set includes a plurality of candidate recommended exercise times. In this case, additional selection may be performed on the plurality of candidate recommended exercise times in the candidate recommended exercise time set to obtain the target recommended exercise time.

In some examples, a plurality of candidate recommended exercise times may be selected from the candidate exercise time set, prompt information is then output to prompt the target object to make a selection from the plurality of selected candidate recommended exercise times, and the target recommended exercise time is determined based on the received input information from the target object. In this way, the selected target recommended exercise time may better meet the need or preference of the target object, so as to improve motivation of the target object to do exercises.

In some other examples, the target recommended exercise time may be finally determined from a plurality of candidate recommended exercise times based on a historical exercise frequency of the target object at each of the plurality of selected candidate recommended exercise times. For instance, an exercise frequency of the target object at each of the plurality of selected candidate recommended exercise times is determined based at least in part on the historical exercise time data of the target object, and the target recommended exercise time is determined from the plurality of selected candidate recommended exercise times according to the exercise frequency of the target object at each of the plurality of selected candidate recommended exercise times.

In some other implementations, the target recommended exercise time may be determined from a plurality of candidate exercise times included in the candidate exercise time set based on a historical exercise frequency of the target object at each of the plurality of candidate exercise times. For example, an exercise frequency of the target object at each of the plurality of candidate exercise times is determined based at least in part on the historical exercise time data of the target object, and the target recommended exercise time is determined from the plurality of candidate exercise times according to the exercise frequency of the target object at each of the plurality of candidate exercise times. The exercise frequency of the target object may be obtained from all of the historical exercise time data, or be obtained from historical exercise time data within a time interval, or be obtained from historical exercise time data meeting a specific condition, which is not limited herein.

For instance, a candidate exercise time (or candidate recommended exercise time) of the target object corresponding to a maximum exercise frequency among the plurality of candidate exercise times (or candidate recommended exercise times) may be determined as the target recommended exercise time. For instance, the target recommended exercise time may be determined from the following formula:

$$Ext_{recommend}(t) = Argmax\big(Frequency(ExtCandidate)\big)$$

where *ExtCandidate* denotes the candidate recommended exercise time set including a plurality of candidate recommended exercise times of the target object, *Frequency*() denotes an exercise frequency statistical function, $Ext_{recommend}$ (*t*) denotes the target recommended exercise time, and *Argmax* denotes an operation of finding a set of arguments for which the function *Frequency*() attains its maximum value.

At S304, information about the target exercise recommendation result is output.

By implementing the embodiments of the present application, the target recommended exercise time of the target object may be determined based on at least part of the historical sleep data and at least part of the historical exercise time data in the historical exercise data of the target object, which is combined with the exercise and sleep habit or preference of the target object to facilitate the target object to maintain a scientific and regular exercise habit, and to establish a foundation for improvement of exercise ability of the target object and achievement of the proactive health goal.

In some implementations of the present application, an initial exercise recommendation result of the target time period is determined according to the first historical exercise data in the historical exercise data, and adjustment processing is performed on the initial exercise recommendation result based at least in part on the historical sleep data (e.g., based on at least part of the historical sleep data), to obtain the target exercise recommendation result.

Referring to FIG. 4, FIG. 4 is another schematic flowchart of the method for exercise recommendation according to some embodiments of the present application. As shown in FIG. 4, the method may include, but is not limited to, S401 to S404.

At S401, historical sleep data and historical exercise data of the target object are acquired by a terminal device.

At S402, an initial exercise recommendation result of a target time period is determined according to first historical exercise data in the historical exercise data.

The first historical exercise data includes exercise data of one or more time periods prior to the target time period. In some examples, an interval between one or more time periods corresponding to exercise data included in the first historical exercise data and the target time period is within a certain time range. That is, the interval between the one or more time periods corresponding to the first historical exercise data and the target time period is relatively small, so as to better reflect recent exercise condition of the target object.

In some implementations, an exercise recommendation strategy for the target time period is determined according to a physiological measurement result of the target object, and the initial exercise recommendation result of the target time period is determined based on the exercise recommendation strategy for the target time period and the first historical exercise data.

In some examples, a current physiological state of the target object may be determined according to the physiological measurement result of the target object, and the initial exercise recommendation result of the target time period is determined based on the current physiological state of the target object. The current physiological state may refer to a physiological state at or before the determination of the initial exercise recommendation result. The physiological measurement result may include one or more of heart rate data, stress data, health data, and/or the like, which is not limited in the embodiments of the present application.

In some examples, the target exercise recommendation result includes a target recommended exercise amount, and accordingly, the exercise recommendation strategy may include one of increasing the exercise amount, decreasing the exercise amount, and maintaining the exercise amount. Alternatively, the exercise recommendation strategy may be further refined into increasing the exercise amount by a specific magnitude, decreasing the exercise amount by a specific magnitude, and/or the like. For instance, the physiological state of the target object is determined according to the physiological measurement result acquired before the determination of the initial exercise recommendation result, and the exercise recommendation strategy is determined according to the physiological state of the target object. The exercise recommendation strategy may be an exercise recommendation strategy for the target time period, or an exercise recommendation strategy for the current exercise recommendation cycle (e.g., the current week) to which the target time period belongs.

In some examples, historical exercise amount data of the target object may be determined according to the first historical exercise data of the target object, and then the initial exercise recommendation result is determined according to the historical exercise amount data and the exercise recommendation strategy. For instance, amount of exercise performed by the target object during each workout session within a specific time interval may be determined according to the first historical exercise data of the target object collected by the terminal device, an average exercise amount of the target object is then obtained based on the amount of exercise performed by the target object during each workout session within a specific time interval and an exercise frequency of the target object within the time interval, and finally, the initial exercise recommendation result of the target time period is determined based on the average exercise amount of the target object and the exercise recommendation strategy. The specific time interval may be, such as, for example, a previous week, previous 21 days, a previous month, or the like.

In some examples, a recommended total exercise amount of the current exercise recommendation cycle may be determined according to the first historical exercise data of the target object and the exercise recommendation strategy, and then the initial recommended exercise amount of the target time period is determined based on the recommended total exercise amount of the current exercise recommendation cycle and at least one of exercise expert knowledge (or exercise physiological knowledge) or exercise preference information (or exercise setting information) of the target object.

In some examples, the first historical exercise data includes exercise data in at least one historical exercise recommendation cycle prior to the current exercise recommendation cycle. An exercise recommendation cycle may correspond to one week, two weeks, or a specific length of time. For instance, the exercise recommendation cycle is one week, and the recommended total exercise amount in the current week may be determined from the following formula:

$$TRIMP(w) = \begin{cases} CTL_{t-1} * (1 - R) + C1 & if\ Decrease\ load \\ CTL_{t-1} * (1 + R) + C1 & if\ Increase\ load \\ CTL_{t-1} & if\ Maintain\ load \end{cases}$$

where $TRIMP(w)$ denotes the recommended total exercise amount in the current week, $CTL_{t-1}$ denotes an average exercise amount of the target object in a previous week, R and C1 denote a climb rate and an offset coefficient, respectively, which may be acquired from prior knowledge of sleep or obtained according to at least one of attribute information, the historical exercise data, or physiological data of the target object, *Decrease load* represents the exercise recommendation strategy being decreasing the exercise amount, *Increase load* represents the exercise recommendation strategy being increasing the exercise amount, and *Maintain load* represents the exercise recommendation strategy being maintaining the exercise amount.

Then, an initial exercise recommendation result for each day of the current week is determined based on the initial recommended exercise time and the recommended total exercise amount of the target object in the current week.

In some examples, the historical exercise data may include motion data and heart rate data. According to the motion data and the heart rate data within a certain time interval included in the first historical exercise data, an average exercise amount of the target object within the time interval may be obtained. For instance, in response to the heart rate data of the target object indicating that a current heart rate value is greater than a preset heart rate threshold (e.g., 110 beats/minute) and the motion data indicating that the current acceleration is greater than a preset acceleration threshold, it is determined that the target object is currently in an exercise state. The amount of exercise performed by the target object during each exercise session is obtained according to a duration during which the target object is in the exercise state and to the heart rate data of the target object in the exercise state. The amount of exercise may alternatively be an exercise impulse or the like, which is not limited in the embodiments of the present application.

At S403, adjustment processing is performed on the initial exercise recommendation result based on at least part of the historical sleep data, to obtain the target exercise recommendation result.

The at least part of the historical sleep data may refer to all or a portion of the historical sleep data, such as, for example, historical sleep data within a certain time interval. In some examples, the adjustment processing may be performed on the initial exercise recommendation result according to sleep data in one or more time periods which are close to the target time period and have a time interval from the target time period less than a set value, such as, for example, sleep data during a previous day, or sleep data in a previous week, or sleep data during one or more days that are passed in the current week, or the like. For instance, the adjustment processing may be performed on the initial exercise recommendation result according to the sleep data during the previous day. For another instance, the adjustment processing is performed on the initial exercise recommendation result according to sleep data in a period of time that is passed in the current exercise recommendation cycle. For yet another instance, the adjustment processing is performed on the initial exercise recommendation result according to sleep data in at least one historical exercise recommendation cycle prior to the current exercise recommendation cycle. For still another instance, the adjustment processing is performed on the initial exercise recommendation result according to sleep data in a period of time that is spaced a specific length of time from the target time period.

In some embodiments, a sleep quality assessment result for the target object is obtained according to the at least part of the historical sleep data; and adjustment processing is performed on the initial exercise recommendation result based on the sleep quality assessment result for the target object, to obtain the target exercise recommendation result.

For instance, a sleep quality of the target object during at least one day prior to the target time period is assessed according to the at least part of the historical sleep data, to obtain the sleep quality assessment result, and the adjustment processing is performed on the initial exercise recommendation result based on the sleep quality assessment result, to obtain the target exercise recommendation result.

The sleep quality assessment result may include one or more sleep quality indexes, such as a sleep quality score, a sleep quality rating (e.g., excellent, good, pass, fail), or the like. Alternatively, the sleep quality assessment result may include a parameter for indicating whether the sleep quality of the target individual meets a standard, e.g., yes or no, or include an index for indicating a sleep recovery degree, or the like. A standard for evaluating the sleep quality may be directed to all users, e.g., a preset fixed standard. Alternatively, the standard for evaluating the sleep quality may be directed to a user group corresponding to a sleep type same as the target object, e.g., the sleep quality assessment result of the target object is determined with regard to the group of users with the same sleep type. Alternatively, the standard for evaluating the sleep quality may be directed to the target object, e.g., the sleep quality assessment result of the target object is determined with regard to historical sleep condition of the target object, or the like. The implementations of the sleep quality assessment are not limited in the embodiments of the present application.

The adjustment processing may be performed on the initial exercise recommendation result based on a sleep quality assessment result for recent one or more days. For example, if the sleep quality assessment result for the target object indicates that the target object has a relatively high sleep quality during the recent one or more days, the recommended exercise amount included in the initial exercise recommendation result may be kept unchanged or appropriately increased. For another example, if the sleep quality assessment result for the target object indicates that the target object has a relatively low sleep quality during the recent one or more days, the recommended exercise amount included in the initial exercise recommendation result may be appropriately decreased, so as to avoid physical discomfort of the target object due to excessive exercise.

In some other embodiments, the adjustment processing is performed on the initial exercise recommendation result according to the at least part of the historical sleep data and the second historical exercise data in the historical exercise data, to obtain the target exercise recommendation result. The second historical exercise data includes exercise data in one or more time periods close to the target time period. Intervals between the one or more time periods corresponding to the second historical exercise data and the target time period are relatively small and may be within a certain interval range. In some examples, the second historical exercise data includes exercise data in a previous time period or previous $N$ time periods of the target time period, such as, for example, exercise data during a previous day or previous $N$ days. In some other examples, the second historical exercise data includes exercise data in one or more time periods within the current exercise recommendation cycle to which the target time period belongs, such as, for example, exercise data during at least one day that is passed within the current exercise recommendation cycle.

In some examples, the adjustment processing is performed on the initial exercise recommendation result according to the sleep data and the exercise data of one or more time periods within the current exercise recommendation cycle and prior to the target time period, to obtain the target exercise recommendation result. In this case, both of the second historical exercise data and the historical sleep data used for the adjustment of the initial exercise recommendation result may correspond to the period of time that is passed in the current exercise recommendation cycle, e.g., one or more days that are passed in the current week. In some other examples, the second historical exercise data used for the adjustment of the initial exercise recommendation result may correspond to a period of time that is passed in the current exercise recommendation cycle, while the historical sleep data used for the adjustment of the initial exercise recommendation result may correspond to a previous time period of the target time period, e.g., a previous day.

In this way, the adjustment processing may be performed on the initial exercise recommendation result according to exercise and sleep conditions of the target object in a recent period of time, to obtain the target exercise recommendation result, so that the target exercise recommendation result is more in line with a recent physiological state and actual requirements of the target object.

For instance, an exercise assessment result of the target object may be obtained according to the second historical exercise data, where the exercise assessment result is used for indicating an exercise accomplishment condition of the target object; a sleep quality assessment result of the target object during the at least one day before the target time period is obtained according to the at least part of the historical sleep data; and adjustment processing is performed on the initial exercise recommendation result according to the exercise assessment result and the sleep quality assessment result, to obtain the target exercise recommendation result.

In some implementations, an actual exercise amount during each of at least one day close to the target time period may be obtained according to the second historical exercise data, and the exercise assessment result is obtained based at least in part on the actual exercise amount. In some examples, the exercise assessment result may be obtained according to the actual exercise amount during each of the at least one day close to the target time period and recommended exercise information corresponding to the each day.

For instance, it is assumed that the second historical exercise data corresponds to at least one day that is passed within the current exercise recommendation cycle, an actual exercise amount of the target object during each of the at least one day that is passed within the current exercise recommendation cycle may be compared with a recommended exercise amount of the target object during the each day, to determine whether the expected exercise amount of the target object during the each day is achieved; and the exercise assessment result for the target object during the at least one day that is passed within the current exercise recommendation cycle is determined according to the determination whether the expected exercise amount of the target object during each of the at least one day that is passed within the current exercise recommendation cycle is achieved.

In some examples, the exercise assessment result may include a total exercise amount accomplishment indicator and/or an exercise amount accomplishment frequency. The total exercise amount accomplishment indicator may indicate whether an expected total exercise amount during at least one day close to the target time period is achieved, such as, for example, whether an actual total exercise amount during the at least one day that is passed within the current exercise recommendation cycle reaches a recommended total exercise amount of the at least one day, or reaches a recommended total exercise amount of the current exercise recommendation cycle. The exercise amount accomplishment frequency may indicate a ratio of the number of days in which an expected exercise amount is achieved during the at least one day close to the target time period to a total number of days corresponding to the at least one day.

For instance, according to an actual exercise amount of the target object during each of the at least one day close to the target time period, an actual total exercise amount of the target object during the at least one day may be determined. According to the recommended total exercise amount of the at least one day and the actual total exercise amount of the at least one day, it is determined whether the expected total exercise amount of the target object during the at least one day is achieved. The recommended total exercise amount of the at least one day may be the target recommended total exercise amount of the at least one day that is obtained by the adjustment according to the embodiments of the present application. Alternatively, the recommended total exercise amount corresponding to the at least one day may be the unadjusted initial recommended total exercise amount of the at least one day, which is not limited herein.

The actual total exercise amount of the at least one day close to the target time period may refer to a cumulative amount of exercise actually performed by the target object during one or more days that are passed within the current exercise recommendation cycle. The recommended total exercise amount corresponding to the at least one day close to the target time period may include a recommended total exercise amount of the current exercise recommendation cycle, or an accumulation of the recommended exercise amount of each of the one or more days that are passed within the current exercise recommendation cycle.

For another instance, according to the actual exercise amount and the recommended exercise amount of the target object during each of the at least one day close to the target time period, an exercise accomplishment condition during the each day may be determined, and the exercise amount accomplishment frequency of the target object during the at least one day is determined according to the exercise accomplishment condition during each of the at least one day.

In some examples, if the actual exercise amount during a day is greater than or equal to the recommended exercise amount for this day, it is determined that the expected exercise amount of this day for the target object is achieved. If the actual exercise amount during a day is less than the recommended exercise amount for this day, it is determined that the expected exercise amount of the target object during this day is not achieved. The determination of the exercise accomplishment condition may be expressed as follows:

$$Achieve(t) = \begin{cases} 1 & if\ TRIMP'(t) \geq TRIMP(t) \\ 0 & if\ TRIMP'(t) < TRIMP(t) \end{cases}$$

where *TRIMP'*(*t*) denotes the actual exercise amount of the target object during a day, *TRIMP*(*t*) denotes the recommended exercise amount of the target object for this day, and *Achieve*(*t*) denotes the exercise accomplishment condition. In the example shown in the above formula, *Achieve*(*t*) = 1 indicates that the expected exercise amount of the day is achieved, and *Achieve*(*t*) = 0 indicates that the expected exercise amount of the day is not achieved.

In some other examples, the number of days in which the expected exercise amount of the target object is achieved during the at least one day close to the target time period may be determined from the following formula:

$$Achieve\_count(t) = \sum_{i=1}^{t} Achieve(i)$$

where *Achieve_count*(*t*) denotes the number of days in which the expected exercise amount of the target object is achieved during the at least one day close to the target time period, and t denotes a total number of days corresponding to the at least one day close to the target time period.

The exercise amount accomplishment frequency may be obtained by dividing the number of days in which the expected exercise amount is achieved by the total number of days corresponding to the at least one day. Alternatively, the number of days in which the expected exercise amount is achieved may be compared with a preset threshold. In some examples, if the number of days in which the expected exercise amount is achieved is greater than the preset threshold, it is determined that the exercise amount accomplishment frequency meets the requirement. Otherwise, it is determined that the exercise amount accomplishment frequency does not meet the requirement.

In some implementations, the adjustment processing is performed on the initial recommended exercise amount according to at least one of the exercise assessment result or the sleep quality assessment result, to obtain the target recommended exercise amount. The exercise assessment result indicates whether the expected total exercise amount is achieved and/or whether the exercise amount accomplishment frequency reaches a preset threshold.

In some examples, in response to the exercise assessment result indicating that the expected total exercise amount is not achieved and the sleep quality assessment result indicating that the sleep quality is lower than a preset quality threshold, the initial recommended exercise amount included in the initial exercise recommendation result may be adjusted to a minimum exercise amount of the target object to obtain the target recommended exercise amount. In this case, the target recommended exercise amount is the minimum exercise amount of the target object. If the exercise assessment result indicates that the expected total exercise amount is not achieved and the sleep quality assessment result indicates that the sleep quality is lower than the preset quality threshold, the initial recommended exercise amount included in the initial exercise recommendation result may be adjusted to the minimum exercise amount of the target object, regardless of whether or not the exercise amount accomplishment frequency reaches a preset frequency threshold.

In some other examples, in response to the exercise assessment result indicating that the expected total exercise amount is not achieved and the sleep quality assessment result indicating that the sleep quality reaches the preset quality threshold, the initial recommended exercise amount included in the initial exercise recommendation result is not adjusted, and the target recommended exercise amount is the same as the initial recommended exercise amount. In this case, if the exercise assessment result indicates that the expected total exercise amount is not achieved and the sleep quality assessment result indicates that the sleep quality reaches the preset quality threshold, the initial recommended exercise amount included in the initial exercise recommendation result may keep unchanged, regardless of whether or not the exercise amount accomplishment frequency reaches the preset frequency threshold.

In some other examples, in response to the exercise assessment result indicating that the expected total exercise amount is achieved and the exercise amount accomplishment frequency reaches the preset frequency threshold, the initial recommended exercise amount included in the initial exercise recommendation result may be adjusted to zero. That is, the target recommended exercise amount is zero. In this case, if the exercise assessment result indicates that the expected total exercise amount is achieved and the exercise amount accomplishment frequency reaches the preset frequency threshold, regardless of whether or not the sleep quality assessment result indicates that the sleep quality reaches the preset quality threshold, the target recommended exercise amount may be adjusted to zero. That is, the target object is not recommended to do exercise in the target time period.

In some other examples, in response to the exercise assessment result indicating that the expected total exercise amount is achieved and the exercise amount accomplishment frequency is lower than the preset frequency threshold, the initial recommended exercise amount included in the initial exercise recommendation result may be adjusted to the minimum exercise amount of the target object, that is, the target recommended exercise amount is the minimum exercise amount of the target object. In this case, if the exercise assessment result indicates that the expected total exercise amount is achieved and the exercise amount accomplishment frequency is lower than the preset frequency threshold, the initial recommended exercise amount included in the initial exercise recommendation result may be adjusted to the minimum exercise amount of the target object, regardless of whether or not the sleep quality assessment result indicates that the sleep quality reaches the preset quality threshold.

In some other examples, in response to the exercise assessment result indicating that the expected total exercise amount is not achieved and the exercise amount accomplishment frequency is lower than the preset frequency threshold, the initial recommended exercise amount included in the initial exercise recommendation result is not adjusted, i.e., keeps unchanged. In this case, the target recommended exercise amount is the same as the initial recommended exercise amount.

In some other examples, in response to the exercise assessment result indicating that the expected total exercise amount is achieved and the exercise amount accomplishment frequency is lower than the preset frequency threshold, the initial recommended exercise amount included in the initial exercise recommendation result may be adjusted to the minimum exercise amount of the target object. In this case, the target recommended exercise amount is determined to be the minimum exercise amount of the target object.

In some other examples, in response to the exercise assessment result indicating that the expected total exercise amount is achieved and the exercise amount accomplishment frequency reaches the preset frequency threshold, the initial recommended exercise amount included in the initial exercise recommendation result may be adjusted to zero. In this case, the target recommended exercise amount is determined to be zero.

In some embodiments, in response to the exercise assessment result indicating that the expected exercise amount is not achieved and the sleep quality assessment result indicating that the sleep quality is lower than the preset quality threshold, the initial recommended exercise amount included in the initial exercise recommendation result is adjusted to the minimum exercise amount of the target object to obtain the target recommended exercise result.

In some embodiments, in response to the exercise assessment result indicating that the expected exercise amount is not achieved and the sleep quality assessment result indicating that the sleep quality reaches the preset quality threshold, the initial recommended exercise amount is determined as the target recommended exercise amount.

In some embodiments, the sleep quality assessment result includes a sleep recovery degree or a sleep recovery index, and alternatively or additionally, the sleep quality assessment result may include other parameters.

In some implementations, sleep HRV (Heart Rate Variability) data of the target object is obtained based at least in part on the historical sleep data; and a sleep recovery index of the target object is obtained based on the sleep HRV data of the target object.

In some implementations, a specific value of the sleep recovery index of the target object may be determined. Alternatively, a quantitative representation of the sleep recovery index of the target object may be determined to reduce algorithm complexity. For example, it may be determined whether an expected sleep recovery degree of the target object is achieved. For another example, a level of the sleep recovery degree of the target object may be determined, such as good, medium, poor, or the like. Specific implementations of the sleep recovery index are not limited in the embodiments of the present application.

The historical sleep data may include heart rate data obtained during sleep monitoring. Sleep HRV data of the target object may be obtained by processing at least part of the heart rate data included in the historical sleep data. The sleep HRV data may include sleep related HRV data in a relatively short period of time and/or in a relatively long period of time. For instance, the sleep HRV data of the target object may include sleep HRV data of the target object during one or more previous days, or include sleep HRV data in a specific time interval. For another instance, the sleep HRV data of the target object may include data obtained by processing the sleep HRV data of the target object during the one or more previous days, or processing the sleep HRV data in the specific time interval. Alternatively, the sleep HRV data of the target object may include sleep HRV data in different time intervals.

In some examples, the sleep HRV data of the target object includes at least one of a short-term sleep HRV parameter, a long-term sleep HRV parameter, or a sleep HRV parameter of a previous day of the target time period.

The short-term sleep HRV parameter may be used for representing characteristics of sleep HRV of the target object within a relatively short time interval, such as, for example, a previous week, previous two weeks, or consecutive several days in the current week. The short-term sleep HRV parameter may include, for example, a baseline HRV. The short-term sleep HRV parameter may be obtained by processing the sleep heart rate data or sleep HRV data within a first time interval. The long-term sleep HRV parameter may be used for representing characteristics of sleep HRV of the target object within a relatively long time interval, such as, for example, previous three weeks, a previous month, or longer. The long-term sleep HRV parameter may include, for example, a normal fluctuation range of HRV. The long-term sleep HRV parameter may be obtained by processing the sleep heart rate data or sleep HRV data within a second time interval. A length of the second time interval may be greater than that of the first time interval, and the length of the first time interval may be greater than that of the target time period.

In some implementations, the short-term sleep HRV parameter may be obtained by processing the sleep HRV data of the target object in a preset time window that is relatively short, such as, for example, by performing averaging processing on the sleep HRV data of the target object. In some examples, a baseline HRV of the target object at the current day or current time point may be obtained from the following formula:

$$Baseline(t) = \frac{1}{T1} \sum_{i=t-T1+1}^{t} HRV(i)$$

where *Baseline*(*t*) denotes the baseline HRV of the target object at the current day or the current time point, *T1* denotes a preset short-term assessment time window, and *HRV*(*i*) denotes sleep HRV data of the *i*th day.

In some examples, the short-term sleep HRV parameter may include only the current baseline HRV, i.e., the baseline HRV at the current day or current time point. Alternatively, the short-term sleep HRV parameter may include the baseline HRV of at least two days. For example, the short-term sleep HRV parameter may include a baseline HRV of the current day and a baseline HRV of the previous day, which is not limited in the embodiments of the present application.

In some implementations, the long-term sleep HRV parameter may be obtained by processing the sleep HRV data of the target object in a preset time window that is relatively long. In some examples, a normal fluctuation range of HRV of the target object at the current day or the current time point may be obtained from the following formula:

$$N\_range(t) = \frac{1}{T2} \sum_{i=t-T2+1}^{t} HRV(i) \pm 0.75 * \sigma$$

where *N_range*(*t*) denotes a normal fluctuation range of HRV at the current day or current time point, *T2* denotes a preset long-term assessment time window, $\sigma$ denotes a standard deviation in the interval of 72, and 0.75 is a fitting parameter which may be adjusted according to actual situations.

In some implementations, by taking the long-term sleep HRV parameter as a reference, the sleep recovery index of the target object may be determined based on at least one of the short-term sleep HRV parameter of the target object or the sleep HRV parameter of the target object in the previous day.

In some examples, the sleep recovery index of the target object may be obtained by comparing at least one of the sleep HRV parameter of the target object in the previous day or the short-term sleep HRV parameter of the target object at the current day with the long-term sleep HRV parameter. For instance, if both the sleep HRV parameter of the target object in the previous day and the baseline HRV of the target object at the current day are within the normal fluctuation range, it may be determined that the sleep recovery index of the target object has a first value, or that the expected sleep recovery degree is achieved. For another instance, if the sleep HRV of the target object during the previous day is not within the normal fluctuation range, it is determined that the sleep recovery index of the target object has a second value less than the first value, or that the expected sleep recovery degree is not achieved. For yet another instance, if the baseline HRV of the target object at the current day exceeds an upper limit of the normal fluctuation range, it is determined that the sleep recovery index of the target object has the first value, or that the expected sleep recovery degree is achieved. For still another instance, if the baseline HRV of the target object at the current day is less than a lower limit of the normal fluctuation range, it is determined that the sleep recovery index of the target object has the second value, or that the expected sleep recovery degree is not achieved.

In some other examples, the sleep recovery index of the target object may be determined by analyzing a change in the short-term sleep HRV parameters of the target object, such as, for example, a change in the baseline HRVs of at least two days. For instance, if the baseline HRV of the target object at the current day is greater than or equal to the baseline HRV of the target object in the previous day, it is determined that a value of the sleep recovery index of the target object is the first value or that the expected sleep recovery degree is achieved.

In some other examples, at least two of the above implementations may be combined to obtain the sleep recovery index of the target object. For instance, if the sleep HRV parameter of the target object of the previous day is not within the normal fluctuation range, and the baseline HRV of the current day exceeds the upper limit of the normal fluctuation range, it is determined that the sleep recovery index of the target object has the first value, or that the expected sleep recovery degree is achieved. For another instance, if the sleep HRV parameter of the target object of the previous day is not within the normal fluctuation range, and the baseline HRV of the current day is greater than the baseline HRV of the previous day, it is determined that the sleep recovery index of the target object has the first value, or that the expected sleep recovery degree is achieved, and so on. Implementations of determination of the sleep recovery index of the target object are not limited in the embodiments of the present application.

In some embodiments, the target recommended exercise amount may be determined by using at least one of the sleep recovery index or the exercise assessment result for the target object. For example, if the expected total exercise amount of the target object in the current week is not achieved, e.g., an total amount of exercise that is actually performed in the current week is less than a recommended total exercise amount in the current week, and the sleep recovery index indicates a relatively high sleep recovery degree, e.g., the sleep recovery index has the first value, then the initial recommended exercise amount is adjusted to the minimum exercise amount of the target object. The minimum exercise amount of the target object may be determined based on at least one of attribute information, the historical exercise data, or the exercise preference information of the target object. For another example, if the expected total exercise amount of the target object in the current week is not achieved, and the sleep recovery index indicates a relatively low sleep recovery degree, e.g., the sleep recovery index has the second value, then the initial recommended exercise amount is not adjusted. For yet another example, if the expected total exercise amount of the target object in the current week is achieved, while the exercise amount accomplishment frequency is relatively low, e.g., the exercise amount accomplishment frequency does not reach the preset frequency threshold, then the recommended exercise amount may be adjusted to the minimum exercise amount of the target object. For still another example, if the expected total exercise amount of the target object has been achieved, and the exercise amount accomplishment frequency is relatively high, e.g., the exercise amount accomplishment frequency reaches the preset frequency threshold, then the recommended exercise amount may be adjusted to zero or other preset values, such as, for example, a value set in advance by the target object.

At S404, information about the target exercise recommendation result is output.

In some embodiments, only the target recommended exercise amount is output. In some examples, if the initial recommended exercise amount is different from the target recommended exercise amount, both the information on the initial recommended exercise amount and the information on the target recommended exercise amount may be output, and the user is prompted to instruct whether to agree with the adjustment of the recommended exercise amount, or to select between the initial recommended exercise amount and the target recommended exercise amount, which is not limited in the embodiments of the present application.

In some other implementations of the present application, the initial exercise recommendation result of the target time period may be determined, and adjustment processing is performed on the initial exercise recommendation result according to the at least part of the historical sleep data and the second historical exercise data in the historical exercise data, to obtain the target exercise recommendation result.

In some examples, the initial exercise recommendation result of the target time period may be determined according to the first historical exercise data with reference to the above embodiments. In some examples, the initial exercise recommendation result of the target time period may be obtained according to first historical exercise data of a time period which is within at least one historical exercise recommendation cycle and corresponds to the target time period. For instance, the actual exercise time and the actual exercise amount included in the first historical exercise data of the corresponding time period are used as the initial recommended exercise time and the initial recommended exercise amount of the target time period, respectively.

In some other examples, the initial exercise recommendation result of the target time period may be determined according to the first historical exercise data and a physiological measurement result for the target object. For instance, a current physiological state of the target object may be obtained according to the physiological measurement result for the target object, and the initial recommended exercise amount and/or the initial recommended exercise time of the target time period are determined according to the current physiological state of the target object.

In some other examples, the initial exercise recommendation result of the target time period may be determined according to user input information. For instance, the user enters an exercise plan for a certain period of time, e.g., a certain day or week. In this case, the initial exercise recommendation result of the target time period may be determined according to the exercise plan entered by the user.

In some other examples, the initial exercise recommendation result of the target time period may be determined according to historical exercise recommendation information within a time interval, such as historical exercise recommendation information within a previous time period, multiple previous time periods, or at least one historical exercise recommendation cycle. For instance, the initial exercise recommendation result of the target time period is determined according to the recommended exercise time and/or the recommended exercise amount of a time period (e.g., a day of the week) that is in the at least one historical exercise recommendation cycle (such as a previous week or multiple previous weeks) and corresponds to the target time period. In some examples, the recommended exercise time in the corresponding time period may be taken as the initial recommended exercise time in the target time period, and the recommended exercise amount in the corresponding time period may be taken as the initial recommended exercise amount in the target time period. Alternatively, the recommended exercise amount in the corresponding time period may be adjusted by a certain amount, for example, according to the current physiological state of the target object, to obtain the initial recommended exercise amount of the target time period. In some other examples, the historical exercise recommendation information and the first historical exercise data may be combined to obtain the initial exercise recommendation result of the target time period. For instance, the historical exercise recommendation information is adjusted according to the first historical exercise data to obtain the initial exercise recommendation result of the target time period.

In some other examples, the initial exercise recommendation result may be obtained according to one or more types of information, such as attribute information (e.g., profile information) of the target object, a user group to which the target object belongs, the sleep type of the target object, or a sleep group to which the target object belongs. For instance, the initial exercise recommendation result of a day or of each day of a week is obtained based on the sleep group to which the target object belongs and exercise physiology knowledge, or based on the sleep type of the target object and the exercise physiology knowledge.

In some other examples, any two or more examples of the implementations described above may be combined to obtain the initial exercise recommendation result of the target time period.

At or before the arrival of the target time period, adjustment processing is performed on the initial exercise recommendation result according to the sleep data of at least one day close to the target time period and the exercise data of the at least one day close to the target time period, to obtain the target exercise recommendation result. For example, adjustment processing is performed on the initial exercise recommendation result according to the exercise data of at least one day in the current exercise recommendation cycle and the sleep data of a previous day of the target time period, to obtain the target exercise recommendation result, specific implementations of which may be found in the above embodiments and will not be repeated herein.

In some examples, a first exercise recommendation for the target object of a plurality of exercise recommendation cycles may be obtained according to at least one of the attribute information of the target object, the exercise preference information of the target object, prior information of exercise, the historical exercise data of the target object, or the physiological measurement result for the target object. The first exercise recommendation of different exercise recommendation cycles may be the same, or may change with a certain step size, or the like. The first exercise recommendation of each of the exercise recommendation cycles may include a plurality of first exercise recommendation results of a plurality of time periods included in the exercise recommendation cycle, or may include only a total exercise recommendation of the exercise recommendation cycle without being further divided for individual time periods. In some optional examples, a first adjustment may be performed on the first exercise recommendation. For instance, before or at arrival of each exercise recommendation cycle, adjustment processing may be performed on the first exercise recommendation of the exercise recommendation cycle according to the historical exercise data of at least one historical exercise recommendation cycle prior to the exercise recommendation cycle, or, adjustment processing may be performed on the first exercise recommendation result of one or more time periods in the exercise recommendation cycle, to obtain a second exercise recommendation of the exercise recommendation cycle, where the second exercise recommendation may include a plurality of second exercise recommendation results of the plurality of time periods included in the exercise recommendation cycle. In some other optional examples, a second adjustment may be performed on the second exercise recommendation. For instance, before or at arrival of each time period in the exercise recommendation cycle, adjustment processing may be performed on the second exercise recommendation result of the time period based on the historical exercise data and/or the historical sleep data of at least one historical time period (e.g., at least one time period that is passed) in the exercise recommendation cycle, to obtain a third exercise recommendation result of the time period. Finally, the user is provided with the third exercise recommendation result of the time period. In some examples, the first exercise recommendation result of a certain time period may be the initial exercise recommendation result of the time period, and the second exercise recommendation result may be the target exercise recommendation result of the time period. In some other examples, the second exercise recommendation result of a certain time period may be the initial exercise recommendation result of the time period, and the third exercise recommendation result may be the target exercise recommendation result of the time period. In some other examples, the first exercise recommendation result of a certain time period may be the initial exercise recommendation result of the time period, and the third exercise recommendation result of the time period may be the target exercise recommendation result of the time period, which is not limited in the embodiments of the present application.

Referring to FIG. 5, FIG. 5 is a schematic diagram of an exemplary implementation for exercise amount recommendation according to some embodiments of the present application.

At S501, exercise monitoring of a target object may be performed to obtain exercise data of the target object.

The exercise monitoring of the target object may be performed by one or more sensors in the wearable device or other types of terminal devices, to obtain the exercise data of the target object, such as, for example, one or more of heart rate data, motion data, location data, an exercise type, an exercise intensity, an exercise start time, an exercise end time, and/or the like.

At S502, sleep monitoring of the target object may be performed to obtain sleep data of the target object.

For example, the sleep monitoring of the target object may be performed by one or more sensors in the wearable device or other types of terminal devices, or by a sleep monitor provided on a bed, to obtain the sleep data of the target object. The sleep data may include one or any combination of a sleep start time, a sleep end time, sleep staging data, sleep heart rate data, sleep respiration data, and/or the like.

At S503, an initial recommended exercise amount of the target object may be obtained by using an exercise recommendation model according to the exercise data of the target object.

For example, the initial recommended exercise amount of the target object during each day in the current exercise recommendation cycle (e.g., one week or other lengths of time) is obtained based on the exercise data of the target object in at least one historical exercise recommendation cycle (e.g., previous few weeks or other lengths of time).

At S504, exercise amount accomplishment frequency detection may be performed according to the exercise data of the target object, to obtain a current exercise amount accomplishment frequency of the target object.

For example, a current exercise amount accomplishment frequency of the target object is obtained by using the exercise data of the target object in the current exercise recommendation cycle.

At S505, a current sleep recovery index of the target object may be obtained by using a sleep recovery assessment model based on the sleep data of the target object.

For example, the current sleep recovery index of the target object may be obtained based on the sleep data of the target object in a previous period of time, e.g., a previous day, a previous week, or a specific length of time.

At S506, adjustment processing is performed on the initial recommended exercise amount of the target time period by using a dynamic adjustment strategy based on at least one of the current exercise amount accomplishment frequency or the current sleep recovery index of the target object, to obtain a target recommended exercise amount of the target time period.

In some examples, whether to adjust the initial recommended exercise amount is determined according to the current sleep recovery index. For instance, if the current sleep recovery index reaches a preset recovery threshold, the initial recommended exercise amount is not adjusted. That is, the target recommended exercise amount is the same as the initial recommended exercise amount. For another instance, if the current sleep recovery index does not reach the preset recovery threshold, the initial recommended exercise amount is determined to be adjusted. In this case, the initial recommended exercise amount may be directly adjusted to the minimum exercise amount of the target object or zero, or, the target recommended exercise amount is determined based on the current exercise amount accomplishment frequency. For instance, if the current exercise amount accomplishment frequency reaches a preset frequency threshold, the initial recommended exercise amount is adjusted to zero. For another instance, if the current exercise amount accomplishment frequency does not reach the preset frequency threshold, the initial recommended exercise amount is adjusted to the minimum exercise amount of the target object.

With the embodiments of the present application, the initial recommended exercise amount of the target object may be determined, and the initial recommended exercise amount may be dynamically adjusted according to at least one of the current exercise condition or sleep condition of the target object, thereby helping the target object maintain a scientific and regular exercise habit, and establishing a foundation for improvement of exercise ability for the target object and achievement of the proactive health goal.

As may be seen from the above mentioned embodiments, with the method for exercise recommendation according to the present application, the target object may be provided with an appropriate exercise recommendation to help the target object do exercise scientifically, which may further has an effect of sleep aid for the target object, thereby improving the sleep quality of the target object at night. Meanwhile, good sleep quality at night can also increase willingness of the target object to exercise during the daytime, making it easier for the target object to accept the exercise recommendation. Finally, if the sleep quality of the target object at night is maintained at a relatively high level, the proactive health goal of the target object will be achieved more easily.

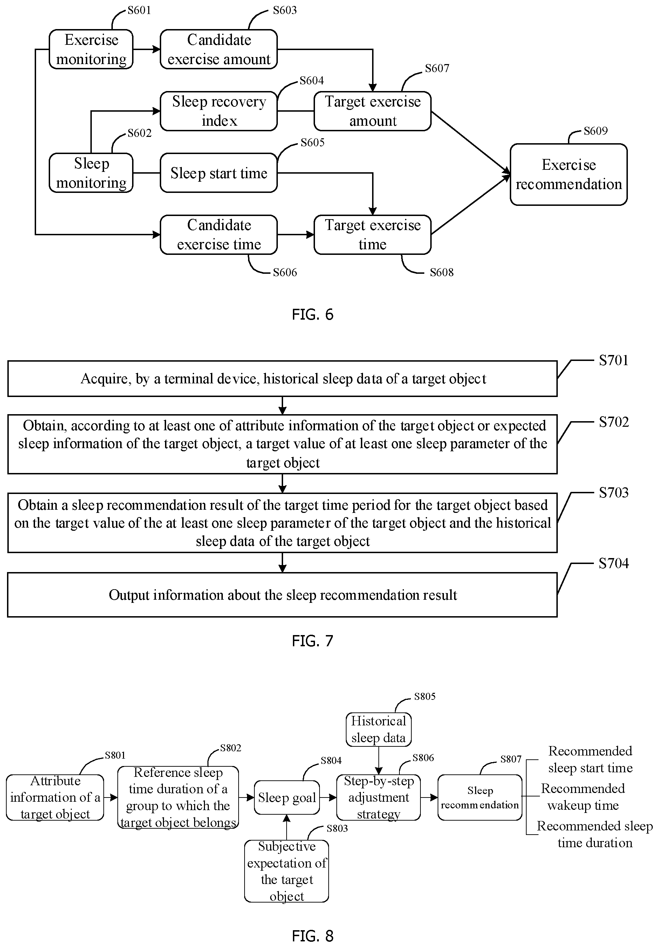

Referring to FIG. 6, FIG. 6 is a schematic diagram of an exemplary implementation of the exercise recommendation according to some embodiments of the present application.

As shown in FIG. 6, the exercise recommendation is based on bidirectional influences between exercise and sleep. At S601, exercise monitoring of a target object is performed by a terminal device such as a wearable device, to obtain exercise data of the target object. At S602, sleep monitoring of the target object is performed by the terminal device such as the wearable device, to obtain sleep data of the target object.

At S603 and S606, a candidate exercise amount and a candidate exercise time of the target object may be determined respectively based on the exercise data of the target object. For example, the candidate exercise amount of the target object may be obtained based on the exercise data of the target object in a previous time interval with a first specific length, and the candidate exercise time of the target object may be obtained based on the exercise data of the target object in a previous time interval with a second specific length. The determination of the candidate exercise amount and the candidate exercise time may rely on exercise data in the same time interval or in different time intervals. For instance, the first specific length may be shorter than the second specific length, but the embodiments of the present application are not limited thereto.

At S604 and S605, a sleep start time and a sleep recovery index of the target object may be determined respectively based on the sleep data of the target object. For example, a current sleep recovery index of the target object may be determined based on the sleep data of the target object in a preceding time interval with a specific length, where the specific length may be the same as or different from a length of the time interval used in the determination of the candidate exercise amount or the candidate exercise time. For another example, the sleep start time (e.g., time to fall asleep or time to go to bed) of the target object is determined based on the sleep data of the target object during a previous day, previous few days or previous few weeks.

At S607, a target exercise amount of the target time period may be determined based on the candidate exercise amount and the current sleep recovery index of the target object.

For example, based on the current sleep recovery index of the target object, at least one candidate exercise amount may be selected from a plurality of candidate exercise amounts as the target exercise amount. For another example, adjustment processing may be performed on one or more candidate exercise amounts based on the current sleep recovery index, to obtain the target exercise amount.

At S608, a target exercise time of the target object may be determined based on the candidate exercise time and the sleep start time of the target object.

For example, based on the sleep start time of the target object, at least one candidate exercise time may be selected from a plurality of candidate exercise times as the target exercise time. For another example, adjustment processing may be performed on one or more candidate exercise times based on the sleep start time of the target object, to obtain the target exercise time, where the adjustment may be narrowing down, deleting, adding, shifting, or the like.

At S609, an exercise recommendation for the target time period may be output. The exercise recommendation may indicate the target exercise time and the target exercise amount of the target object in the target time period. For example, information about the exercise recommendation may be sent to the wearable device, or the information about the exercise recommendation may be directly output to the user, or the information about the exercise recommendation may be sent to other devices, which is not limited in the embodiments of the present application.

In some embodiments of the present application, the target object may be further provided with a sleep recommendation based on the sleep data of the target object and expected sleep information of the target object.

In some examples, referring to FIG. 7, FIG. 7 is a schematic flowchart of a method for sleep recommendation according to some embodiments of the present application. As shown in FIG. 7, the method may include, but is not limited to, S701 to S704.

At S701, historical sleep data of a target object is acquired by a terminal device.

The historical sleep data of the target object may be collected by the terminal device. The terminal device may collect sleep data by at least one of a motion sensor or a physiological sensor. The motion sensor includes, for example, one or more sensors such as an accelerometer and a gyroscope. The physiological sensor may include, for example, one or more of a heart rate sensor, a respiration sensor, a body temperature sensor, a blood pressure sensor, and/or the like.

The historical sleep data may include sleep data in one or more time periods prior to the target time period. In some examples, the historical sleep data may include sleep data during one or more days prior to the target time period. For instance, the historical sleep data includes sleep data during a previous day. In some other examples, the historical sleep data includes sleep data during one or more days within a current sleep recommendation cycle, such as, for example, sleep data of one or more days that are in the current week and prior to the current day. In some other examples, the historical sleep data includes sleep data in at least one historical sleep recommendation cycle, such as, for example, sleep data during a plurality of days in a previous week. In some other examples, the historical sleep data may be the historical sleep data in the above mentioned embodiments, which is not limited in the embodiments of the present application.

The historical sleep data may include one or any combination of a sleep start time (e.g., sleep onset time), a sleep end time (e.g., wakeup time), a sleep time duration, a number of awakenings, a cumulative awakening time duration, sleep respiration data, sleep heart rate data, sleep latency, and/or the like. The specific implementations may be found in the above mentioned embodiments, and will not be repeated herein.

At S702, a target value of at least one sleep parameter of the target object is obtained according to at least one of attribute information of the target object or expected sleep information of the target object.

In some examples, the attribute information of the target object includes personal profile information of the target object, such as one or any combination of age, gender, BMI, occupation, hobby, sleep habit, and geographical location. In some other examples, the attribute information of the target object may include other information, such as, for example, one or any combination of: a user group to which the target object belongs, a sleep group to which the target object belongs, a sleep type of the target object, and/or the like, which is not limited in the embodiments of the present application.

In some examples, the expected sleep information of the target object includes an expected value of the at least one sleep parameter, i.e., an expected value of one or more sleep parameters, such as, for example, one or any combination of an expected sleep duration, an expected wakeup time, and an expected sleep start time, or alternatively includes expected values of other sleep parameters. The expected sleep information may be obtained from received user input information, or may be obtained according to sleep information of a group to which the target object belongs, or the like.

In some other examples, the expected sleep information may include one or more sleep improvement aspects for the target object, such as, for example, a longer sleep time duration, an increasing deep sleep ratio, a shorter sleep latency, or the like.

The target value of the at least one sleep parameter of the target object may include a target value of one or more sleep parameters, such as, for example, one or any combination of a target sleep start time, a target wakeup time, a target sleep time duration, and/or the like.

In some examples, the target value of the at least one sleep parameter of the target object is obtained according to the attribute information of the target object. For instance, the target value of the at least one sleep parameter of the target object is obtained according to a user group and/or a sleep group to which the target object belongs.

In some other examples, the target value of the at least one sleep parameter of the target object is obtained according to the expected sleep information of the target object. For instance, the expected value of the at least one sleep parameter included in the expected sleep information is determined as the target value of the at least one sleep parameter. For another instance, the target value of the at least one sleep parameter is obtained by processing the expected value of the at least one sleep parameter included in the expected sleep information.

In some other examples, the target value of the at least one sleep parameter of the target object is obtained according to the expected sleep information of the target object and the attribute information of the target object. For instance, some of the target values of the sleep parameters is determined to be corresponding expected values of the sleep parameters in the expected sleep information, and the rest of the target values of the sleep parameters is obtained based on one or more expected values of the sleep parameters included in the expected sleep information or the determined target values of the sleep parameters, as well as on the attribute information. For another instance, a weight for the expected sleep information and a weight for the attribute information may be set, and the target value of the at least one sleep parameter is obtained according to the expected sleep information, the attribute information, and respective weights thereof. For another instance, the target value of the at least one sleep parameter may be obtained by processing the expected sleep information and the attribute information with a sleep improvement model. For another instance, the target value of the at least one sleep parameter may be obtained in other manners.

In some other examples, it is assumed that the attribute information of the target object and the expected sleep information of the target object are both acquired or both are available, one of the attribute information and the expected sleep information of the target object is considered as a preference factor to obtain the target value of the at least one sleep parameter of the target object. For instance, the expected sleep information is considered as the preference factor. In this case, if there is available expected sleep information, the target value of the at least one sleep parameter is obtained based on the available expected sleep information, and if no expected sleep information is available, the target value of the at least one sleep parameter is obtained based on the attribute information. For another instance, the attribute information is considered as the preference factor. In this case, if there is available attribute information, an initial value of the at least one sleep parameter is obtained based on the available attribute information, and the initial value of the at least one sleep parameter is adjusted based on the expected sleep information, to obtain the target value of the at least one sleep parameter.

In some implementations, a validity of the expected sleep information may be firstly judged, and a manner in which the target value of the at least one sleep parameter is obtained may be determined based on a validity judgment result. In this case, the target value of the at least one sleep parameter may be determined based on the validity judgment result of the expected sleep information, as well as at least one of the expected sleep information or the attribute information.

In some examples, a validity of each of some or all of the expected values of the sleep parameters included in the expected sleep information may be determined. For instance, it is determined whether each of the expected values of the sleep parameters is within a corresponding preset range. Different preset ranges may be set for expected values of different sleep parameters. If the expected value of a sleep parameter is within a corresponding preset range, it is determined that the expected value of the sleep parameter is valid; and otherwise, it is determined that the expected value of the sleep parameter is invalid. The preset range may be determined according to prior knowledge of sleep, or, the preset range may be determined according to the attribute information of the target object, such as, for example, a group to which the target object belongs. Alternatively, the preset range is determined in other manners, which is not limited herein.

In this case, in some optional examples, if a certain sleep parameter has a valid expected value, that is, the expected sleep information includes a valid expected value of the sleep parameter, the expected value of the sleep parameter may be used to determine a target value of the sleep parameter, i.e., the target value corresponding to the sleep parameter. For instance, the valid expected value of the sleep parameter is determined as the target value of the sleep parameter. For another instance, a corresponding target value of the sleep parameter is obtained by processing the valid expected value of the sleep parameter.

In some other optional examples, if a certain sleep parameter does not have a valid expected value, that is, the expected sleep information does not include a valid expected value of the sleep parameter, for example, the expected sleep information does not include an expected value of the sleep parameter, or the expected value of the sleep parameter included in the expected sleep information is not within a corresponding preset range, then the target value of the sleep parameter may be determined based on at least one of the attribute information of the target object, a valid expected value or a corresponding target value of another sleep parameter, or physiology knowledge of sleep.

In some other examples, a validity of the expected sleep information may be judged as a whole. In this case, a validity judgment result of the expected sleep information may include: an indication of the expected sleep information being valid, or an indication of the expected sleep information being invalid. For instance, it may be judged whether the expected sleep information is valid by determining whether the expected value of the at least one sleep parameter included in the expected sleep information is valid. In some examples, if all of the one or more expected values of the at least one sleep parameter included in the expected sleep information are invalid, or the number or a proportion of invalid expected values of the at least one sleep parameter included in the expected sleep information exceeds a certain threshold, it is determined that the expected sleep information is invalid. For another instance, it is judged whether the expected sleep information is valid by determining whether the expected sleep information includes an expected value of a certain sleep parameter, or determining whether an expected value of a certain sleep parameter included in the expected sleep information is valid. For another instance, if the expected sleep information includes expected values of a plurality of sleep parameters, it may be judged whether the expected sleep information is valid by determining whether a value of a sleep parameter (e.g., sleep time duration) obtained by calculating expected values of the plurality of sleep parameters is within a reasonable range. For another instance, it may be judged whether the expected sleep information is valid by determining whether expected values of the plurality of the sleep parameters are self-consistent, e.g., whether a time interval between an expected sleep start time and an expected sleep end time is equal to an expected sleep time duration, or the like. The implementations for judging the validity of the expected sleep information is not limited herein.

In this case, in some optional examples, in response to the validity judgment result of the expected sleep information indicating that the expected sleep information is valid, the target value of the at least one sleep parameter is obtained by using the expected value of the at least one sleep parameter included in the expected sleep information. In some other optional examples, in response to the validity judgment result indicating that the expected sleep information is invalid, the target value of the at least one sleep parameter is determined by using the attribute information.

In some implementations, the target value of the at least one sleep parameter may be determined based on a validity judgment result of the expected value of the at least one sleep parameter. In some examples, it may be judged whether the expected sleep information includes a valid expected value corresponding to a certain sleep parameter. If a valid expected value corresponding to the sleep parameter is included in the expected sleep information, a target value corresponding to the sleep parameter may be obtained by using the valid expected value of the sleep parameter. If the expected sleep information does not include a valid expected value corresponding to the sleep parameter, the target value corresponding to the sleep parameter may be determined by using the attribute information of the target object and/or prior knowledge of sleep.

In some examples, the target value of the at least one sleep parameter includes a target sleep time duration. In this case, it may be judged whether the expected sleep information includes a valid expected sleep time duration. If the expected sleep information includes a valid expected sleep time duration, the target sleep time duration of the target object is determined as the valid expected sleep time duration. If the expected sleep information does not include a valid expected sleep time duration, for example, the expected sleep information does not include the expected sleep time duration, or the expected sleep time duration included in the expected sleep information is invalid, the target sleep time duration is determined based on the attribute information of the target object. For example, the target sleep time duration of the target object is determined based on at least one of gender, age, or BMI of the target object, as well as on prior knowledge of sleep and/or a preset mapping rule. For another example, a group (e.g., a user group and/or a sleep group) to which the target object belongs is determined based on the attribute information of the target object, and the target sleep time duration of the target object is determined based on information of the group to which the target object belongs.

In some examples, the target sleep time duration may be obtained from the following formula:

$$TargetTST = \begin{cases} TargetTST_{user} & \text{if } TargetTST_{user} \text{ is valid} \\ TargetTST_{rule} & \text{if } TargetTST_{user} \text{ is NOT valid} \end{cases}$$

where *TargetTST* denotes the target sleep time duration, *TargetTST$_{rule}$* denotes a recommended sleep time duration for the group to which the target object belongs, and *TargetTST$_{user}$* denotes an expected sleep time duration included in the expected sleep information of the target object.

In this example, if the expected sleep time duration of the target object included in the expected sleep information is valid, the target sleep time duration is determined to be the expected sleep time duration. If the expected sleep time duration included in the expected sleep information is not valid, the target sleep time duration is determined to be the recommended sleep time duration for the group to which the target object belongs.

In some other embodiments, if the expected sleep information does not include a valid expected value of a certain sleep parameter, a target value of the sleep parameter may be determined by using the target values of other sleep parameters and/or valid expected values of other sleep parameters included in the expected sleep information. In some examples, some of target values of the sleep parameters may be determined according to at least one of the attribute information or the expected sleep information of the target object, and the rest of the target values of the sleep parameters are determined based on at least one of the determined target values of the sleep parameters or the expected sleep information of the target object. For instance, the target value of the at least one sleep parameter includes a target sleep time duration and a target time to fall asleep (or a target sleep start time) and/or a target wakeup time (or a target sleep end time), and the target sleep start time may be determined based on the target sleep time duration and/or a valid expected wakeup time included in the expected sleep information. For another instance, the target wakeup time may be determined based on the target sleep time duration and/or a valid expected sleep start time included in the expected sleep information.

In some examples, the target sleep start time may be determined from the following formula:

$$TargetST = \begin{cases} TargetST_{rule} & if\ TargetST_{user}\ is\ None\ AND\ TargetWT_{user}\ is\ None \\ TargetST_{user} & if\ TargetST_{user}\ is\ Not\ None \\ TargetWT_{user} - TargetTST & if\ TargetWT_{user}\ is\ Not\ None \end{cases}$$

**where** $TargetST$ denotes the target sleep start time, $TargetST_{rule}$ denotes a sleep start time determined according to the attribute information of the target object and/or prior knowledge of sleep, $TargetST_{user}$ denotes the expected sleep start time of the target object, $TargetWT_{user}$ denotes the expected wakeup time of the target object, and $TargetTST$ denotes the target sleep time duration.

In this example, if the expected sleep information includes an expected sleep start time (or a valid expected sleep start time), the target sleep start time is determined to be the expected sleep start time. If the expected sleep information includes an expected wakeup time (or a valid expected wakeup time), the target sleep start time may be determined according to the expected wakeup time and the target sleep time duration. If the expected sleep information includes neither the expected sleep start time (or the valid expected sleep start time), nor the expected wakeup time (or the valid expected wakeup time), the target sleep start time is determined according to the attribute information of the target object and/or prior knowledge of sleep. For instance, the target sleep start time of the target object is determined to be the recommended sleep start time for the group to which the target object belongs.

In some examples, the target sleep time duration and the target sleep start time may be determined respectively by using the above formulas, and the target wakeup time of the target object may be determined according to the target sleep time duration and the target sleep start time.

For instance, the target wakeup time $TargetWT$ *may* be determined from the following formula:

$$TargetWT = \begin{cases} TargetST_{rule} + TargetTST & if\ TargetST_{user}\ AND\ TargetWT_{user}\ are\ None \\ TargetST_{user} + TargetTST & if\ TargetST_{user}\ is\ Not\ None \\ TargetWT_{user} & if\ TargetWT_{user}\ is\ Not\ None \end{cases}$$

**where** $TargetST_{rule}$ denotes the sleep start time determined according to the attribute information of the target object and/or prior knowledge of sleep, $TargetTST$ denotes the target sleep time duration, $TargetST_{user}$ denotes the expected sleep start time of the target object, and $TargetWT_{user}$ denotes the expected wakeup time of the target object.

In this example, if the expected sleep information includes an expected wakeup time (or a valid expected wakeup time), the target wakeup time is determined to be the expected wakeup time. If the expected sleep information includes an expected sleep start time (or a valid expected sleep start time), the target wakeup time is obtained according to the expected sleep start time and the target sleep time duration. If the expected sleep information of the target object includes neither the expected sleep start time (or the valid expected sleep start time), nor the expected wakeup time (or the valid expected wakeup time), the target wakeup time is determined based on the target sleep time duration and the target sleep start time determined according to the attribute information of the target object and/or prior knowledge of sleep.

It may be understood by those skilled in the art that the above mentioned implementations of the target sleep start time and the target wakeup time may alternatively be interchangeable or determined in other manners, which is not limited in the embodiments of the present application.

At S703, a sleep recommendation result of the target time period for the target object is obtained based on the target value of the at least one sleep parameter and the historical sleep data of the target object.

In some embodiments of the present application, the sleep recommendation result includes a recommended value of the at least one sleep parameter. For example, the sleep recommendation result includes at least one of a recommended sleep start time, a recommended wakeup time, or a recommended sleep time duration.

The historical sleep data may include sleep data in one or more time periods prior to the target time period, and the historical sleep data may be used for indicating recent sleep condition of the target object, specific implementations of which may be found in the above mentioned embodiments. In some examples, the historical sleep data may include sleep data of a previous week or a previous month, or sleep data of other time intervals, which is not limited herein.

In some examples, a current value of the at least one sleep parameter of the target object is obtained based on the historical sleep data of the target object, and the current value of the at least one sleep parameter of the target object is adjusted according to a preset sleep adjustment strategy based on the target value of the at least one sleep parameter of the target object, to obtain the recommended value of at least one sleep parameter included in the sleep recommendation result for the target object. In some examples, the preset sleep adjustment strategy may include a progressive adjustment strategy (or referred to as a step-by-step adjustment strategy) or a direct adjustment strategy (or referred to as a single-step adjustment strategy or a one-step adjustment strategy). In the progressive adjustment strategy, the current value of the sleep parameter may be used as a starting point to gradually approach the target value of the sleep parameter over time. In the direct adjustment strategy, the current value of the sleep parameter may be directly adjusted to the target value of the sleep parameter. In some other examples, the preset sleep adjustment strategy may be implemented in other manners, which is not limited herein.

It should be noted that the specific implementations of the target time period may be found in the above embodiments. The sleep recommendation result of the target time period may include a sleep recommendation result for the current day, the next day, the next week, or a certain time interval defined by the target object.

At S704, information about the sleep recommendation result is output.

S701 and S702 may be performed in any sequence or simultaneously. In some examples, S702 may be performed first and the target value of the at least one sleep parameter may be stored, and then S701 may be performed. In this case, the historical sleep data of the target object is collected by the terminal device, the stored target value of the at least one sleep parameter is acquired, and finally, the sleep recommendation result of the target time period is obtained based on the historical sleep data and the target value of the at least one sleep parameter. In some other examples, S701 may be performed first. In this case, the historical sleep data of the target object is collected by the terminal device and then stored, and then S702 is performed to obtain the target value of the at least one sleep parameter of the target object, which is not limited herein.

With the embodiments of the present application, the target value of the at least one sleep parameter of the target object may be obtained according to at least one of the attribute information or the expected sleep information of the target object, and the sleep recommendation result for the target object is obtained based on the target value of the at least one sleep parameter and the historical sleep data of the target object, so as to help the target object gradually establish a scientific and regular sleep habit.

Referring to FIG. 8, FIG. 8 is a schematic diagram of an exemplary implementation of the sleep recommendation according to some embodiments of the present application.

At S801, attribute information of the target object may be acquired, where the attribute information of the target object includes user profile information, such as one or any combination of age, gender, BMI, and/or the like.

At S802, a reference sleep time duration of a group to which the target object belongs may be determined based on the attribute information of the target object.

For example, the group to which the target object belongs may be determined based on the attribute information of the target object, and the reference sleep time duration is determined according to the group to which the target object belongs, such as attribute information of the group and sleep information of other individuals in the group.

At S803, subjective expectation information of the target object may be acquired. For example, the subjective expectation information input by the target object is acquired through a user interaction operation, and includes, for example, an expected value of one or more sleep parameters such as one or any combination of an expected sleep start time, an expected sleep time duration, and an expected wakeup time. Optionally, the subjective expectation information may be the expected sleep information in the above embodiments.

At S804, a sleep goal for the target object may be determined based on at least one of the subjective expectation information of the target object or the reference sleep time duration of the group to which the target object belongs. The sleep goal may include a target value of one or more sleep parameters such as one or any combination of a target sleep start time, a target wakeup time, and a target sleep time duration.

In some examples, if it is determined that the expected sleep time duration included in the subjective expectation information is valid, S802 may not be performed, which is not limited in the embodiments of the present application.

At S805, sleep monitoring is performed for the target object to obtain the historical sleep data of the target object within a preset time interval. The historical sleep data may include one or any combination of a sleep start time, a sleep end time, sleep staging data, sleep respiration data, sleep quality data, and/or the like. In this example, the preset time interval may be a previous week, which is not limited in the embodiments of the present application.

At S806, a sleep recommendation (which is output at S807) of the target time period for the target object may be obtained by using the step-by-step adjustment strategy based on the sleep goal for the target object and the historical sleep data of the target object in the preset time interval. The sleep recommendation includes a recommended value of at least one sleep parameter such as one or any combination of a recommended sleep start time, a recommended sleep time duration, and a recommended wakeup time, so as to help the target object gradually establish a regular sleep habit.

In some implementations, a sleep adjustment strategy for the target object is determined based on at least part of the historical sleep data of the target object and the target value of the at least one sleep parameter, and a sleep recommendation result for the target object is obtained according to the sleep adjustment strategy.

In some examples, the sleep adjustment strategy may include a progressive adjustment strategy or a direct adjustment strategy. The progressive adjustment strategy is to gradually adjust the sleep parameter of the target object with a small stride. The direct adjustment strategy is to adjust the sleep parameter of the target object to the target value at one time.

In some implementations, the sleep adjustment strategy for the target object may be determined based on a difference between current value of the at least one sleep parameter indicated by the historical sleep data and target value of the at least one sleep parameter. In some examples, if a difference between the current value of the at least one sleep parameter indicated by the historical sleep data and the corresponding target value of the at least one sleep parameter is relatively small, such as, for example, within a preset difference range, the direct adjustment strategy may be adopted. In some other examples, if a difference between the current value of the at least one sleep parameter indicated by the historical sleep data and the corresponding target value of the at least one sleep parameter is relatively large, such as, for example, exceeds a preset difference range, the progressive adjustment strategy may be adopted to cause the sleep parameter of the target object to gradually achieve the sleep goal.

In some implementations, the historical sleep data of the target object may be processed, statistically for example, to obtain the current value of the at least one sleep parameter of the target object. For instance, the historical sleep data of the target object within a certain time interval may be processed to obtain an average sleep time duration of the target object within the time interval. The average sleep time duration may be obtained by averaging the sleep time duration during a plurality of days within the time interval, e.g., the average sleep duration is an average value of the sleep time duration during the plurality of days, or the average sleep time duration may be obtained by averaging the sleep time duration during the plurality of days after filtering out the maximum value, the minimum value, or an outlier value therefrom, or the average sleep time duration may be obtained by performing statistical processing on the sleep time duration with frequencies of occurrence exceeding a certain threshold, or the like, which is not limited in the embodiments of the present application.

In some examples, in response to a difference between a current value of the at least one sleep parameter indicated by the historical sleep data of the target object and the target value of the at least one sleep parameter exceeding a preset difference range, the sleep recommendation result for the target object is obtained based on an adjustment step size and the current value of the at least one sleep parameter.

For instance, the current value of the at least one sleep parameter is adjusted with the adjustment step size to obtain the recommended value of the at least one sleep parameter included in the sleep recommendation result, so that the recommended value of the at least one sleep parameter included in the sleep recommendation result gradually approaches the target value of the at least one sleep parameter over time. In this case, the current value of the at least one sleep parameter may be adjusted by using the progressive adjustment strategy.

The adjustment step size may be set in advance. Alternatively, the adjustment step size may be obtained according to the attribute information of the target object, such as, for example, according to a group to which the target object belongs. Alternatively, the adjustment step size may be obtained according to the difference between the current value of the at least one sleep parameter and the target value of the at least one sleep parameter. For instance, the adjustment step size is obtained based on the difference and a time interval for achieving the sleep goal of the target object. Alternatively, the adjustment step size is determined according to user input information, or the like. Implementations of the adjustment step size are not limited herein.

In some other examples, in response to the difference between the current value of the at least one sleep parameter indicated by the historical sleep data of the target object and the target value of the at least one sleep parameter being within the preset difference range, the recommended value of the at least one sleep parameter included in the sleep recommendation result for the target object is determined to be the target value of the at least one sleep parameter. In this case, the current value of the at least one sleep parameter may be adjusted by using the direct adjustment strategy.

In some implementations, the difference between the current value of the at least one sleep parameter and the target value of the at least one sleep parameter may include a difference between each of the current values of a plurality of sleep parameters and a corresponding target value of the sleep parameter. In this case, the difference between the current value of the at least one sleep parameter and the target value of the at least one sleep parameter being within the preset difference range may indicate that, each of the differences is within a corresponding preset difference range, or a proportion of the differences that is within the corresponding preset difference range exceeds a certain value, or a statistical result of the differences is within the preset difference range, or the like. Alternatively, the difference between the current value of the at least one sleep parameter and the target value of the at least one sleep parameter includes a difference between the current value of a certain sleep parameter and the corresponding target value of the sleep parameter. Alternatively, the difference between the current value of the at least one sleep parameter and the target value of the at least one sleep parameter includes statistical result of one or more differences between the current value of the at least one sleep parameter and the target value of the at least one sleep parameter, such as the maximum difference, the minimum difference, or an average difference, which is not limited herein.

In some examples, assuming that the historical sleep data includes the sleep data of a previous week, and the sleep recommendation result of the target time period includes a recommended sleep time duration and a recommended sleep start time of the current week, the recommended sleep time duration of the current week may be obtained from the following formula:

$$TST_{recommend}(w) = \begin{cases} TargetTST & if\ TargetTST - TST(w-1) \le C5 \\ TST(w-1) + \Delta TST & if\ TargetTST - TST(w-1) > C5 \end{cases}$$

where $TST_{recommend}(w)$ denotes the recommended sleep time duration of the current week, C5 denotes a preset difference range corresponding to the sleep time duration, $\Delta TST$ **denotes an** adjustment step size corresponding to the sleep time duration, e.g., twenty minutes, and $TST(w-1)$ denotes an average sleep time duration of the target object in the previous week.

In this example, the average sleep time duration of the target object in the previous week is taken as a current sleep time duration of the target object. If a difference between the average sleep time duration of the target object in the previous week and the target sleep time duration is less than or equal to C5, the direct adjustment strategy is adopted to determine the recommended sleep time duration in the current week as the target sleep time duration. If the difference between the average sleep time duration of the target object in the previous week and the target sleep time duration is greater than C5, the progressive adjustment strategy is adopted to determine the recommended sleep time duration in the current week as an increase or decrease in units of $\Delta TST$ from the average sleep time duration in the previous week. The adjustment step size $\Delta TST$ may be a preset fixed value, or may be determined based on an expectation of the target object, or determined based on the attribute information of the target object, or the like. In some examples, the adjustment step size $\Delta TST$ corresponding to the sleep time duration may be equal to the preset difference range C5 corresponding to the sleep time duration. In some other examples, the adjustment step size $\Delta TST$ corresponding to the sleep time duration may alternatively be set to other values different from the preset difference range C5 corresponding to the sleep time duration, which is not limited in the embodiments of the present application.

In some other examples, the recommended sleep start time in the current week may be obtained from the following formula:

$$ST_{recommend}(w) = \begin{cases} TargetST & if\ TargetST - ST(w-1) \le C6 \\ ST(w-1) + \Delta ST & if\ TargetST - ST(w-1) > C6 \end{cases}$$

where $ST_{recommend}(w)$ denotes the recommended sleep start time in the current week, $ST(w-1)$ denotes an average sleep start time of the target object in the previous week, $\Delta ST$ denotes an adjustment step size corresponding to the sleep start time, and C6 denotes a preset difference range corresponding to the sleep start time.

In this example, the current sleep start time of the target object is determined to be the average sleep start time of the target object in the previous week. If a difference between the average sleep start time of the target object in the previous week and the target sleep start time is less than or equal to C6, the direct adjustment strategy is adopted to determine the recommended sleep start time in the current week as the target sleep start time. If the difference between the average sleep start time of the target object in the previous week and the target sleep start time is greater than C6, the progressive adjustment strategy is adopted to determine the recommended sleep start time in the current week as an increase or decrease in units of $\Delta ST$ from the average sleep start time in the previous week. The adjustment step size $\Delta ST$ may be a preset fixed value, or determined based on an expectation of the target object, or determined based on the attribute information of the target object, or the like. In some examples, the adjustment step size $\Delta ST$ corresponding to the sleep start time may be equal to the preset difference range C6 corresponding to the sleep start time. In some other examples, the adjustment step size $\Delta ST$ corresponding to the sleep start time may alternatively be set to other values different from the preset difference range C6 corresponding to the sleep start time, which is not limited in the embodiments of the present application.

In some implementations of embodiments of the present application, guidance on supplemental sleep of the target object may also be provided. A supplemental sleep recommendation result of the target time period for the target object may be obtained based on the historical sleep data of the target object collected by the terminal device. The supplemental sleep recommendation result may include a recommendation for the target object to take a supplemental sleep or not, or may include a recommended time duration for the supplemental sleep, the maximum sleep time duration for the supplemental sleep, the latest wakeup time for the supplemental sleep, or the latest sleep time for the target object to take the supplemental sleep, and/or the like, which is not limited in the embodiments of the present application.

In the present disclosure, the term "regular sleep" may refer to sleep for a relatively long time that the target object engages in, such as night-time sleep, while the term "supplemental sleep" may refer to sleep for a relatively short time that the target object engages in, such as daytime nap or midday nap.

In some implementations, the supplemental sleep recommendation result of the target time period may be obtained according to sleep data during a previous day or according to sleep data during one or more days that are passed in the current week.

In some examples, the physiological data and body movement data of the target object during the regular sleep may be measured to obtain regular sleep data of the target object during the previous day, such as a time duration of the regular sleep, quality assessment of the regular sleep, and/or staging data of the regular sleep, and the supplemental sleep recommendation result of the target time period for the target object is obtained based on the regular sleep data. In some examples, if the time duration of the regular sleep during the previous day is less than a preset time duration threshold, the target object may be recommended to take a supplemental sleep in the target time period. In this case, the supplemental sleep recommendation result may include a first indicator for recommending to take a supplemental sleep. Alternatively, the supplemental sleep recommendation result includes a recommended time duration of the supplemental sleep, which is a first time duration greater than zero. In some other examples, if the time duration of regular sleep during the previous day is equal to or greater than the preset time duration threshold, the target object may be recommended not to take a supplemental sleep in the target time period. In this case, the supplemental sleep recommendation result may include a second indicator for recommending no supplemental sleep, or, the recommended time duration of supplemental sleep included in the supplemental sleep recommendation result is zero. In some other examples, if the time duration of regular sleep during the previous day is equal to or greater than the preset time duration threshold, it may be determined whether to recommend supplemental sleep in the target time period based on historical supplemental sleep data included in the historical sleep data of the target object. For instance, if the historical supplemental sleep data of the target object indicates that the target object has a habit of taking a supplemental sleep, the target object may be recommended to take a supplemental sleep in the target time period. If the historical supplemental sleep data of the target object indicates that the target object has no habit of taking a supplemental sleep, the target object may be recommended not to take a supplemental sleep in the target time period.

The preset time duration threshold may be a preset fixed value, e.g., 8 hours, or may be determined based at least in part on the historical sleep data of the target object, e.g., an average time duration of regular sleep of the target object, or may be determined according to expected sleep information or a sleep goal of the target object, which is not limited herein.

In some other examples, it may be determined whether to recommend a supplemental sleep in the target time period according to a quality assessment result of regular sleep for the target object during the previous day. For instance, it may be determined whether to recommend supplemental sleep in the target time period according to a sleep recovery index of the target object, or, it may be determined whether to recommend a supplemental sleep in the target time period based on other types of historical sleep data, which is not limited herein.

In some examples, in the case that supplemental sleep is recommended, a recommended time duration of the supplemental sleep may also be determined. The recommended time duration of the supplemental sleep may be preset, e.g., 30 minutes or no more than 1 hour. Alternatively, the recommended time duration of the supplemental sleep may be determined based on subjective expectation information of the target object and a time duration of the regular sleep during the previous day. Alternatively, the recommended time duration of the supplemental sleep may be determined according to the historical supplemental sleep data included in the historical sleep data of the target object, e.g., an average time duration of the supplemental sleep in a previous week. Alternatively, the recommended time duration of the supplemental sleep may be determined according to the attribute information of the target object, or the like.

In some examples, the recommended time duration of the supplemental sleep in the target time period may be determined from the following formula:

$$Nap_{\text{recommend}}(t) = \begin{cases} C7 \ if \ TST(t-1) < TST_{threshold} \\ 0 \ if \ TST(t-1) \geq TST_{threshold} \end{cases}$$

where $Nap_{recommend}(t)$ denotes the recommended time duration of supplemental sleep in the target time period, $TST(t-1)$ denotes the time duration of regular sleep of the target object during the previous day, $TST_{threshold}$ denotes a sleep time duration threshold, and C7 denotes the first time duration, which may be a preset fixed value, or obtained according to the attribute information of the target object, or obtained based at least in part on the historical sleep data of the target object.

In some other examples, the supplemental sleep recommendation result for the target object is obtained according to at least one of regular sleep data of the target object during the previous day or supplemental sleep data of the target object during the previous day.

In some other examples, in the case that the target object is recommended to take a supplemental sleep in the target time period, the supplemental sleep recommendation result may further include a warning for the time duration of supplemental sleep, such as, for example, warning not to take supplemental sleep for too long, or reminding to set an alarm before taking the supplemental sleep, or the like, so as to avoid the subsequent regular sleep or mental state of the target object in the daytime to be influenced due to taking too long supplemental sleep.

In some examples, the warning for time duration of supplemental sleep for the target object may be obtained based on the historical sleep data of the target object. For instance, it may be determined whether to generate the warning for time duration of supplemental sleep based on the historical supplemental sleep data in the historical sleep data. For instance, in response to the historical supplemental sleep data indicating that the time duration of supplemental sleep for the target object during the previous day exceeds a recommended time duration, the warning for time duration of supplemental sleep is generated to remind the target object that the time duration of supplemental sleep in the target time period should not exceed the recommended time duration.

In some examples, the warning for time duration of supplemental sleep may be obtained from the following formula:

$$Nap\ Warning(t) = \begin{cases} True & if\ Nap'(t) > Nap_{recommend} \\ False & if\ Nap'(t) \leq Nap_{recommend} \end{cases}$$

where $Nap\ Warning(t) = True$ denotes that a warning for time duration of supplemental sleep should be generated, $Nap\ Warning(t) = False$ denotes that the warning for time duration of supplemental sleep should not be generated, $Nap_{recommend}$ denotes the recommended time duration of supplemental sleep, and $Nap'(t)$ denotes the time duration of supplemental sleep for the target object during the previous day.

In this example, in response to the historical supplemental sleep data of the target object indicating that the time duration of supplemental sleep for the target object during the previous day exceeds the recommended time duration, the warning for time duration of supplemental sleep is output to remind the target object to appropriately adjust the time duration of supplemental sleep; and otherwise, no warning for time duration of supplemental sleep is output.

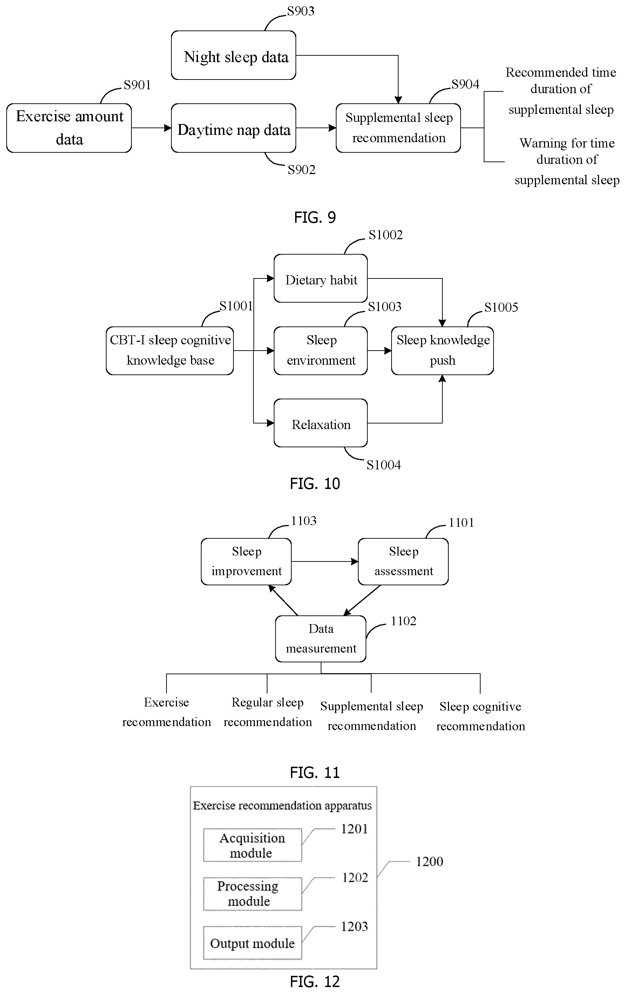

Referring to FIG. 9, FIG. 9 is a schematic diagram of an exemplary implementation of supplemental sleep recommendation according to some embodiments of the present application.

At S901, historical exercise data of the target object, such as, for example, exercise amount data during a previous day, may be acquired.

At S902, historical supplemental sleep data of the target object, such as, for example, daytime nap data during the previous day, may be acquired.

At S903, historical regular sleep data of the target object, such as, for example, night sleep data during the previous day, may be acquired.

At S904, a supplemental sleep recommendation for the target object is obtained based on the data obtained in the S901~S903.

In some examples, a time duration of supplemental sleep of the target object during the previous day may be monitored. If the time duration of supplemental sleep during the previous day is greater than a preset time duration threshold, or greater than a recommended time duration of supplemental sleep, a warning for time duration of supplemental sleep may also be provided in the supplemental sleep recommendation, so as to prevent the target object from taking too long supplemental sleep in the target time period.

With the embodiments of the present application, the supplemental sleep recommendation result for the target object may be obtained based on the historical sleep data of the target object, thereby preventing the target object from suffering from daytime fatigue due to poor night sleep and also avoiding adverse effects of relatively long supplemental sleep on health, so as to establish a foundation for achievement of the proactive health goal of the target object.

In some embodiments of the present application, sleep health of the target object may be further improved by providing the target object with appropriate sleep cognitive information.

Referring to FIG. 10, FIG. 10 is a schematic diagram of an exemplary implementation of sleep recommendation according to some embodiments of the present application. At S1001, sleep cognitive knowledge for the target object may be acquired from a CBT-I (Cognitive Behavioral Therapy for Insomnia) sleep cognitive knowledge base based on attribute information of the target object.

At S1002, a dietary recommendation for the target object is obtained according to the acquired sleep cognitive knowledge.

At S1003, a sleep environment recommendation for the target object is obtained according to the acquired sleep cognitive knowledge.

At S1004, a relaxation recommendation for the target object is obtained according to the acquired sleep cognitive knowledge.

At S1005, at least one of the recommendations obtained in S1002~S1004 is pushed to the target object.

In some examples, the sleep cognitive information may include at least one of the dietary recommendation, the sleep environment recommendation, or the relaxation recommendation. The dietary recommendation may include that, for example, if you are sensitive to stimulant drinks such as coffee or tea, please avoid drinking these drinks after 2:00 p.m. The sleep environment recommendation may include that, for example, an appropriate temperature allows people to fall asleep faster, and please keep a generally low bedroom temperature at about 21°C to 24°C. The sleep environment recommendation may include that, for another example, the optimal bed temperature is 1°C to 2°C higher than the surface temperature of a human body. The relaxation recommendation may include that, for example, do something to make yourself quiet and relaxed before going to bed, such as meditation or book reading.

Such sleep cognitive information may be universal or may be determined based on characteristics of the target object, such as, for example, based on the attribute information of the target object, e.g., one or more of age, gender, hobby, living habit, and/or the like, or based on a current health state of the target object, e.g., one or more of a sleep type, an exercise habit type, disease information, and/or the like, or based on historical monitoring information of the target object, e.g., one or more of coffee intake beyond a preset time point, an excessive sleep environment temperature, a relatively high level of stress before going to bed, and/or the like, which is not limited in the embodiments of the present application.

By implementing the embodiments of the present application, the target object may be provided with the sleep cognitive information, so as to further help the target object improve the sleep quality, thereby helping the target object develop a healthy sleep habit.

In some embodiments, the electronic device may make both exercise recommendation and sleep recommendation based at least in part on the historical sleep data and the historical exercise data of the target object.

Referring to FIG. 11, FIG. 11 is a schematic diagram of an exemplary health guidance system according to some embodiments of the present application. As shown in FIG. 11, the health guidance system mainly includes a sleep assessment module 1101, a data measurement module 1102, and a sleep improvement module 1103.

The sleep assessment module 1101 may be configured to assess a sleep quality of the target object. For example, a sleep quality assessment result and/or a current value of at least one sleep parameter of the target object may be obtained according to the sleep data obtained by the data measurement module 1102, to indicate a current sleep condition of the target object.

The data measurement module 1102 may be configured to measure and process data of the target object. For example, the data of the target object, such as sleep data (e.g., regular sleep data and/or supplemental sleep data), exercise data, and physiological measurement data, is collected and processed by a terminal device.

The sleep improvement module 1103 may be configured to calculate a path to achieve a sleep goal, and obtain a sleep improvement recommendation based on the current physiological or sleep condition of the target object and the sleep issue which currently exists or is expected to be improved.

In some examples, the sleep improvement recommendation may include one or any combination of an exercise recommendation, a regular sleep recommendation, a supplemental sleep recommendation, and a sleep cognitive recommendation.

In some examples, the sleep improvement recommendation may include the target exercise recommendation result described in the above embodiments.

In some other examples, the sleep improvement recommendation may include the sleep recommendation result described in the above embodiments.

In some other examples, the sleep improvement recommendation may include the target exercise recommendation result and the sleep recommendation result.

It can be seen that, in the present application, under the framework of proactive health management, a closed loop of proactive health guidance is formed by combining exercise and sleep aspects of the individual. Based on the historical exercise and sleep data of the target object and in conjunction with prior knowledge of sleep, operational improvement schemes are provided for the target object to achieve the health goal.

Referring to FIG. 12, FIG. 12 is a schematic diagram of an apparatus for exercise recommendation 1200 according to some embodiments of the present application. As shown in FIG. 12, the apparatus 1200 includes: an acquisition module 1201 configured to acquire, by a terminal device, historical sleep data and historical exercise data of a target object, the historical sleep data including sleep data during at least one day prior to a target time period, and the historical exercise data including exercise data during at least one day prior to the target time period; a processing module 1202 configured to obtain, according to the historical sleep data and the historical exercise data, a target exercise recommendation result of the target time period for the target object, the target exercise recommendation result including at least one of a target recommended exercise time or a target recommended exercise amount; and an output module 1203 configured to output information about the target exercise recommendation result.

In some embodiments, the time point when the procedure is performed is referred to as a current day, and the target exercise recommendation result of the target time period for the target object includes a target exercise recommendation result for the target object during the following day, such as, for example, a target exercise recommendation result of the current day (i.e., today) or a target exercise recommendation result of the next day (i.e., tomorrow).

In some embodiments, the processing module 1202 is configured to: determine a candidate exercise time set based at least in part on historical exercise time data included in the historical exercise data, the candidate exercise time set including at least one candidate exercise time; and determine the target recommended exercise time from the candidate exercise time set based at least in part on the historical sleep data.

The candidate exercise time set may include one or more candidate exercise time points or periods. The one or more candidate exercise time points or periods may be determined according to part or all of the historical exercise time data included in the historical exercise data. For example, all exercise time points or periods included in the historical exercise time data may be taken as the candidate exercise time points or periods. Alternatively, one or more exercise time points or periods among all the exercise time points or periods included in the historical exercise time data that meet a certain condition may be taken as the candidate exercise time points or periods. For example, one or more exercise time points or periods in the historical exercise time data whose number of occurrence reaches a certain threshold are taken as the candidate exercise time points or periods, and so on. Alternatively, the candidate exercise time points or periods are obtained by processing part or all of the historical exercise time data.

In some embodiments, the processing module 1202 is configured to: determine a sleep type of the target object, determine an exercise time offset corresponding to the sleep type of the target object from a plurality of preset exercise time offsets, and determine the target recommended exercise time from the candidate exercise time set according to the corresponding exercise time offset and at least part of historical sleep start time data included in the historical sleep data.

In some implementations, the sleep type of the target object may include one of sleep early, sleep late, wakeup early, wakeup late, and insomnia.

In some embodiments, the processing module 1202 is configured to: determine an exercise time requirement based on at least part of the historical sleep start time data included in the historical sleep data and the exercise time offset corresponding to the sleep type of the target object, and determine at least one candidate exercise time in the candidate exercise time set that meets the exercise time requirement as the target recommended exercise time.

In some embodiments, the processing module 1202 is configured to: select a plurality of candidate recommended exercise time points or periods from the candidate exercise time set based at least in part on the historical sleep data, output prompt information to prompt selection from the plurality of candidate recommended exercise time points or periods, and determine the target recommended exercise time based on the received user input information.

In some embodiments, the processing module 1202 is configured to: select a plurality of candidate recommended exercise time points or periods from the candidate exercise time set based at least in part on the historical sleep data, determine an exercise frequency of the target object at each of the plurality of candidate recommended exercise time points or periods according to the historical exercise data, and determine the target recommended exercise time from the plurality of candidate recommended exercise time points or periods according to the exercise frequency of the target object at each of the plurality of candidate recommended exercise time points or periods.

In some embodiments, the processing module 1202 is configured to: determine an initial exercise recommendation result of the target time period according to first historical exercise data in the historical exercise data, and perform adjustment processing on the initial exercise recommendation result based at least in part on the historical sleep data, to obtain the target exercise recommendation result of the target time period.

In some embodiments, the processing module 1202 is configured to: determine an exercise recommendation strategy for the target time period according to a physiological measurement result of the target object; and determine the initial exercise recommendation result of the target time period based on the exercise recommendation strategy for the target time period and the first historical exercise data.

In some embodiments, the processing module 1202 is configured to: obtain a sleep quality assessment result for the target object during at least one day close to the target time period according to the at least part of the historical sleep data, and perform adjustment processing on the initial exercise recommendation result based on the sleep quality assessment result to obtain the target exercise recommendation result.

In some embodiments, the processing module 1202 is further configured to: perform adjustment processing on the initial exercise recommendation result according to the at least part of the historical sleep data of the target object and second historical exercise data in the historical exercise data, to obtain the target exercise recommendation result, where the second historical exercise data includes exercise data during at least one day within a current exercise recommendation cycle and prior to the target time period.

In some embodiments, the processing module 1202 is further configured to: obtain a sleep quality assessment result for the target object during the at least one day prior to the target time period according to the at least part of the historical sleep data of the target object, and perform adjustment processing on the initial exercise recommendation result according to the sleep quality assessment result and the second historical exercise data, to obtain the target exercise recommendation result.

In some implementations, the processing module 1202 is further configured to: obtain an exercise assessment result for the target object according to the second historical exercise data, the exercise assessment result being used for indicating an exercise accomplishment condition of the target object during the at least one day close to the target time period, and perform adjustment processing on the initial exercise recommendation result according to the exercise assessment result and the sleep quality assessment result, to obtain the target exercise recommendation result.

The exercise assessment result includes at least one of an exercise amount accomplishment frequency or a total exercise amount accomplishment indicator. The total exercise amount accomplishment indicator may be used for indicating whether an expected total exercise amount of the target object during the at least one day close to the target time period is achieved. The exercise amount accomplishment frequency may be used for indicating a frequency that an expected exercise amount is achieved during the at least one day, e.g., a ratio of a number of days in which the expected exercise amount is achieved to a total number of days corresponding to the at least one day.

In some implementations, the second historical exercise data includes an actual exercise amount during each of the at least one day close to the target time period. The exercise assessment result is used for indicating an exercise accomplishment condition.

In some implementations, the processing module 1202 is further configured to: obtain the exercise assessment result according to an actual exercise amount during each of the at least one day close to the target time period and recommended exercise information corresponding to the at least one day.

In some implementations, the sleep quality assessment result includes a sleep recovery index. The processing module 1202 is configured to: obtain sleep HRV data of the target object based at least in part on the historical sleep data, and obtain the sleep recovery index of the target object based on the sleep HRV data of the target object.

In some implementations, the apparatus for exercise recommendation provided in the embodiments of the present application includes one or more modules configured to implement steps and/or processes in the above embodiments of the method for exercise recommendation, which will not be repeated herein for brevity.

With the apparatus for exercise recommendation according to the embodiments of the present application, the target exercise recommendation result of the target time period for the target object may be acquired based on the historical sleep data and the historical exercise data of the target object, and the target exercise recommendation result may be recommended to the target object, thereby helping the target object maintain a scientific and regular exercise habit, and establishing a foundation for improvement of exercise capability of the target object and achievement of the proactive health goal.

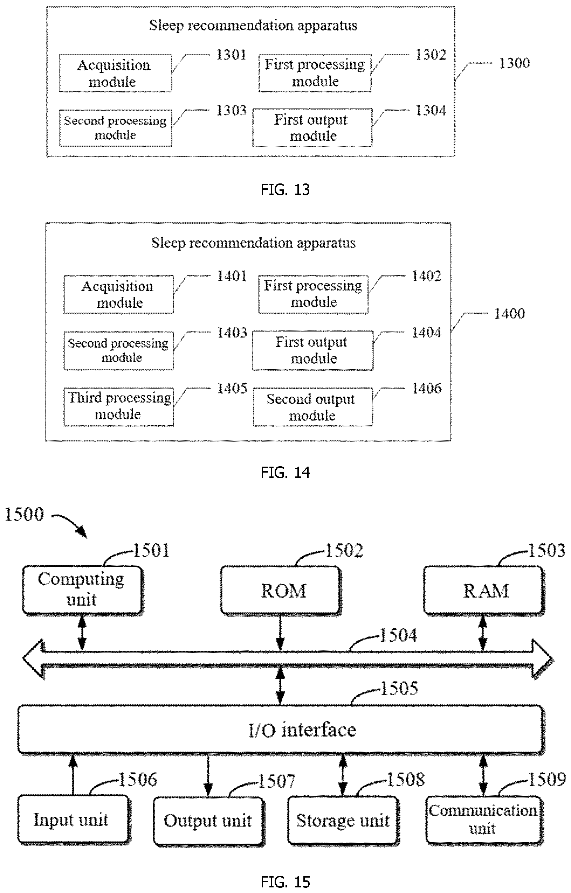

Referring to FIG. 13, FIG. 13 is a schematic diagram of an apparatus for sleep recommendation 1300 according to some embodiments of the present application. As shown in FIG. 13, the apparatus 1300 includes: an acquisition module 1301 configured to acquire, by a terminal device, historical sleep data of a target object; a first processing module 1302 configured to obtain a target value of at least one sleep parameter of the target object according to at least one of attribute information of the target object or expected sleep information of the target object; a second processing module 1303 configured to obtain a sleep recommendation result of a target time period for the target object based on the target value of the at least one sleep parameter of the target object and the historical sleep data of the target object, the sleep recommendation result including at least one of a recommended sleep start time, a recommended wakeup time, or a recommended sleep time duration; and a first output module 1304 configured to output information about the sleep recommendation result.

In some embodiments, the first processing module 1302 is further configured to: determine a validity of an expected value of the at least one sleep parameter included in the expected sleep information before the target value of the at least one sleep parameter is obtained. For example, it is determined whether the expected value of the at least one sleep parameter is within a preset range.

In some embodiments, the first processing module 1302 is configured to: in response to the expected sleep information of the target object including a valid expected value of a first sleep parameter, determine the valid expected value of the first sleep parameter as a target value of the first sleep parameter.

In some embodiments, the first processing module 1302 is configured to: in response to the expected sleep information of the target object not including a valid expected value of a second sleep parameter, determine a target value of the second sleep parameter according to the attribute information of the target object.

In some embodiments, the second processing module 1303 is configured to: determine a sleep adjustment strategy for the target object based on at least part of the historical sleep data of the target object and the target value of the at least one sleep parameter, and obtain the sleep recommendation result of the target time period for the target object according to the sleep adjustment strategy.

The sleep adjustment strategy may include a progressive adjustment strategy or a direct adjustment strategy.

In some embodiments, the second processing module 1303 is further configured to: obtain a current value of the at least one sleep parameter of the target object based at least in part on the historical sleep data of the target object, and obtain the sleep recommendation result according to the current value of the at least one sleep parameter and the target value of the at least one sleep parameter.

The current value of the at least one sleep parameter may include at least one of a historical sleep start time, a historical wakeup time, or a historical sleep time duration.

In some embodiments, the second processing module 1303 is configured to: in response to a difference between the current value of the at least one sleep parameter and the target value of the at least one sleep parameter exceeding a preset difference range, determine the sleep adjustment strategy for the target object to be the progressive adjustment strategy.

In some other embodiments, the second processing module 1303 is configured to: in response to the difference between the current value of the at least one sleep parameter and the target value of the at least one sleep parameter being within the preset difference range, determine the sleep adjustment strategy to be the direct adjustment strategy.

In some embodiments, the apparatus further includes a third processing module 1405. In some examples, referring to FIG. 14, FIG. 14 is a schematic structural diagram of another apparatus for sleep recommendation 1400 according to some embodiments of the present application. As shown in FIG. 14, the apparatus 1400 further includes a third processing module 1405 and a second output module 1406. The third processing module 1405 is configured to obtain a supplemental sleep recommendation result of the target time period for the target object based at least in part on regular sleep data during a previous day of the target time period included in the historical sleep data of the target object. Modules 1401 to 1404 in FIG. 14 have similar structures and functions as the modules 1301 to 1304 in FIG. 13.

In some embodiments, the third processing module 1405 is further configured to: determine whether to recommend the target object to take a supplemental sleep in the target time period according to a determination whether a regular sleep parameter of the target object during the previous day of the target time period reaches a preset parameter range.

In some implementations, the apparatus 1400 further includes the second output module 1406, configured to output a warning for time duration of supplemental sleep based at least in part on supplemental sleep data of the target object during the previous day of the target time period.

In some embodiments, the second output module 1406 is further configured to: in response to the sleep data of the previous day of the target time period indicating that a time duration of supplemental sleep of the target object during the previous day exceeds a recommended time duration range, output the warning for time duration of supplemental sleep to prompt the target object to control the time duration of supplemental sleep in the target time period to be within the recommended time duration range.

In some implementations, the apparatus for sleep recommendation provided in the embodiments of the present application includes one or more modules configured to implement the steps and/or processes in the above embodiments of the method for sleep recommendation, which will not be repeated herein for brevity.

In the apparatus for sleep recommendation according to the embodiments of the present application, the target value of the at least one sleep parameter of the target object may be obtained according to at least one of the attribute information or the expected sleep information of the target object, and the sleep recommendation result of the target time period for the target object may be obtained based on the target value of the at least one sleep parameter of the target object and the historical sleep data of the target object, so as to help the target object gradually establish a scientific and regular sleep habit.

In addition, the supplemental sleep recommendation result for the target object may be obtained based on the historical sleep data of the target object, thereby preventing the target object suffering from mental fatigue due to poor regular sleep and also avoiding adverse effect of relatively long supplemental sleep on health, so as to establish a foundation for achievement of the proactive health goal of the target object.

In the apparatuses in the above embodiments, details described in the above embodiments of the method may be referred to for specific manners in which the modules perform various operations, which will not be described in detail herein.

It should be noted that, acquisition, storage, application, and/or the like of information about the target object involved in the technical solutions of the present application all comply with relevant laws and regulations, and do not violate public order and moral.

Based on the embodiments of the present application, the present application further provides an electronic device, including: at least one processor, and a memory communicatively coupled to the at least one processor, where the memory stores instructions executable by the at least one processor, and execution of the instructions by the at least one processor enables the at least one processor to perform the method for exercise recommendation according to any one of the foregoing embodiments, or enables the at least one processor to perform the method for sleep recommendation according to any one of the foregoing embodiments.

Based on the embodiments of the present application, the present application further provides a computer-readable storage medium, in which computer instructions are configured to cause a computer to perform the method for exercise recommendation according to any one of the foregoing embodiments, or to perform the method for sleep recommendation according to any one of the foregoing embodiments.

Referring to FIG. 15, FIG. 15 is a schematic block diagram of an exemplary electronic device configured to implement the embodiments of the present application. The electronic device is intended to represent various forms of digital computers, such as laptop computers, desktop computers, workbenches, personal digital assistants, servers, blade servers, mainframe computers, and other suitable computers. The components, their connections and relationships, and their functions shown herein are exemplary only, and are not intended to limit implementations of the present application as described and/or claimed herein.

As shown in FIG. 15, the electronic device 1500 includes a computing unit 1501, which may perform various appropriate actions and processes according to a computer program stored in a read-only memory (ROM) 1502 or a computer program loaded from a storage unit 1508 into a random access memory (RAM) 1503. Various programs and data required for operations of the electronic device 1500 may also be stored in the RAM 1503. The computing unit 1501, the ROM 1502, and the RAM 1503 are connected to one another via a bus 1504. An input/output (I/O) interface 1505 is also connected to the bus 1504.

A plurality of components in the electronic device 1500 are connected to the I/O interface 1505, and include: an input unit 1506, such as a keyboard and a mouse; an output unit 1507, such as various types of displays and speakers; the storage unit 1508, such as magnetic disks and optical disks; and a communication unit 1509, such as a network card, a modem, and a wireless communication transceiver. The communication unit 1509 allows the electronic device 1500 to exchange information/data with other devices over a computer network such as Internet and/or various telecommunication networks.

The computing unit 1501 may be a variety of general-purpose and/or special-purpose processing components with processing and computing capabilities. Some examples of the computing unit 1501 include, but are not limited to, a Central Processing Unit (CPU), a Graphics Processing Unit (GPU), a variety of dedicated artificial intelligence (AI) computing chips, a variety of computing units that run machine learning model algorithms, a digital signal processor (DSP), and any appropriate processor, controller, microcontroller, and/or the like. The computing unit 1501 performs the methods and processes as described above, such as the method for exercise recommendation or the method for sleep recommendation. For example, in some embodiments, the method for exercise recommendation or the method for sleep recommendation may be implemented as a computer software program that is tangibly embodied in a machine-readable medium, such as the storage unit 1508. In some embodiments, part or all of a computer program may be loaded and/or installed on the electronic device 1500 via the ROM 1502 and/or the communication unit 1509. One or more steps of the method for exercise recommendation or the method for sleep recommendation as described above may be performed when the computer program is loaded into the RAM 1503 and executed by the computing unit 1501. Alternatively, in some other embodiments, the computing unit 1501 may be configured to perform the method for exercise recommendation or the method for sleep recommendation by any other appropriate means (for example, by means of firmware).

Various embodiments of the systems and technologies described above herein may be implemented in a digital electronic circuit system, an integrated circuit system, a Field Programmable Gate Array (FPGA), an Application Specific Integrated Circuit (ASIC), Application Specific Standard Parts (ASSPs), a System On Chip (SOC), a Complex Programmable Logic Device (CPLD), computer hardware, firmware, software, and/or combinations thereof. These various embodiments may include implementation in one or more computer programs that are executable and/or interpretable on a programmable system including at least one programmable processor, which may be a special-purpose or general-purpose programmable processor that may receive data and instructions from a storage system, at least one input apparatus, and at least one output apparatus, and transmit the data and the instructions to the storage system, the at least one input apparatus, and the at least one output apparatus.

Program codes configured to implement the methods in the present application may be written in any combination of one or more programming languages. Such program codes may be supplied to a processor or controller of a general-purpose computer, a special-purpose computer, or another programmable data processing apparatus to enable the functions/operations specified in the flowchart and/or block diagram to be implemented when the program codes are executed by the processor or controller. The program codes may be executed entirely on a machine, partially on a machine, partially on a machine and partially on a remote machine as a stand-alone software package, or entirely on a remote machine or a server.

In the context of the present application, the machine-readable medium may be a tangible medium, which may include or store programs for use by or in conjunction with an instruction execution system, apparatus, or device. The machine-readable medium may be a machine-readable signal medium or machine-readable storage medium. The machine-readable medium may include, but is not limited to, an electronic, magnetic, optical, electromagnetic, infrared, or semiconductor system, apparatus or device, or any suitable combination thereof. More specific examples of the machine-readable storage medium may include electrical connections based on one or more wires, a portable computer disk, a hard disk, an RAM, an ROM, an Erasable Programmable Read-Only Memory (EPROM or flash memory), optical fiber, a Compact Disc Read-Only Memory (CD-ROM), an optical storage device, a magnetic storage device, or any suitable combination thereof.

To provide interaction with a user, the systems and technologies described herein may be implemented on a computer, which has: a display apparatus (e.g., a Cathode Ray Tube (CRT) or a Liquid Crystal Display (LCD) monitor) for displaying information to the user; and a keyboard and a pointing apparatus (e.g., a mouse or trackball) through which the user may provide input to the computer. Other kinds of apparatuses may be configured to provide interaction with the user, for example, a feedback provided to the user may be any form of sensory feedback (e.g., visual, auditory, or haptic feedback); and the input from the user may be received in any form, including sound input, voice input, or haptic input.

The systems and technologies described herein may be implemented in a computing system including back-end components (e.g., as data servers), or a computing system including middleware components (e.g., application servers), or a computing system including front-end components (e.g., user computers with a graphical user interface or web browser through which the user can interact with the implementation of the systems and technologies described herein), or a computing system including any combination of such back-end components, middleware components, or front-end components. The components of the system may be connected to each other through any form or medium of digital data communication, e.g., a communication network. Examples of the communication network include a Local Area Network (LAN), a Wide Area Network (WAN), Internet, and a blockchain network.

The computer system may include a client and a server. The client and the server are generally remote from each other and generally interact over the communication network. A client-server relationship is created through computer programs that run on corresponding computers and have the client-server relationship with each other. The server may be a cloud server, also known as a cloud computing server or cloud host, which is a host product in a cloud computing service system, solving the defects of difficult management and weak business scalability existing in a traditional physical host and Virtual Private Server (VPS) services. The server may alternatively be a distributed system server, or a server combined with blockchain.

It should be understood that the steps may be reordered, added, or deleted using various forms of the processes shown above. For example, the steps described in the present application may be performed in parallel or sequentially or in different orders, provided that expected results of the technical solutions of the present application may be achieved, which is not limited herein.

The above specific embodiments do not limit the scope of protection of the present application. It should be appreciated by those skilled in the art that various modifications, combinations, sub-combinations, and substitutions may be made according to design requirements and other factors. Any modifications, equivalent substitutions, and improvements made within the spirit and principle of the present application shall be included in the scope of protection of the present application.

**Claims**

1. A method for exercise recommendation, comprising:

   acquiring, by a terminal device, historical sleep data and historical exercise data of a target object, the historical sleep data comprising sleep data of at least one day prior to a target time period, and the historical exercise data comprising exercise data of at least one day prior to the target time period;
   obtaining a target exercise recommendation result of the target time period for the target object based on the historical sleep data and the historical exercise data, the target exercise recommendation result comprising at least one of a target recommended exercise time or a target recommended exercise amount; and
   outputting information about the target exercise recommendation result.

2. The method of claim 1, wherein obtaining the target exercise recommendation result of the target time period for the target object based on the historical sleep data and the historical exercise data, comprises:

   determining a candidate exercise time set based at least in part on historical exercise time data comprised in the historical exercise data, the candidate exercise time set comprising at least one candidate exercise time; and
   determining the target recommended exercise time from the candidate exercise time set based on the historical sleep data.

3. The method of claim 2, wherein determining the target recommended exercise time from the candidate exercise time set based on the historical sleep data, comprises:
   determining the target recommended exercise time from the candidate exercise time set based on a sleep type of the target object and the historical sleep data.

4. The method of claim 2 or 3, wherein determining the target recommended exercise time from the candidate exercise time set based on the historical sleep data, comprises:

   determining, based on a sleep type of the target object, an exercise time offset corresponding to the sleep type of the target object;
   determining an exercise time requirement based on the exercise time offset and at least part of historical sleep start time data comprised in the historical sleep data, the exercise time requirement comprising a latest exercise time; and

44

determining the target recommended exercise time from one or more candidate exercise times in the candidate exercise time set that meet the exercise time requirement.

5. The method of claim 3 or 4, wherein the sleep type comprises at least one of wakeup early, wakeup late, sleep early, sleep late, or insomnia.

6. The method of any one of claims 2 to 5, wherein determining the target recommended exercise time from the candidate exercise time set based on the historical sleep data, comprises:
determining the target recommended exercise time from the at least one candidate exercise time based on the historical sleep data and an exercise frequency corresponding to the at least one candidate exercise time.

7. The method of any one of claims 1 to 6, wherein obtaining the target exercise recommendation result of the target time period for the target object based on the historical sleep data and the historical exercise data, comprises:

determining an initial exercise recommendation result of the target time period based on first historical exercise data in the historical exercise data; and
performing adjustment processing on the initial exercise recommendation result of the target time period based on at least part of the historical sleep data, to obtain the target exercise recommendation result of the target time period for the target object.

8. The method of claim 7, wherein the first historical exercise data comprises exercise data in at least one historical exercise recommendation cycle prior to a current exercise recommendation cycle to which the target time period belongs; and
the at least part of the historical sleep data comprises sleep data during at least one day close to the target time period.

9. The method of any one of claims 1 to 8, wherein obtaining the target exercise recommendation result of the target time period for the target object based on the historical sleep data and the historical exercise data, comprises:

determining, at a first time point, an initial exercise recommendation result of the target time period based on first historical exercise data in the historical exercise data, the first time point being a time point prior to a current exercise recommendation cycle to which the target time period belongs; and
adjusting, at a second time point, the initial exercise recommendation result of the target time period based on at least part of the historical sleep data, to obtain the target exercise recommendation result of the target time period for the target object, the second time point being a time point within the current exercise recommendation cycle and prior to the target time period.

10. The method of any one of claims 7 to 9, wherein determining the initial exercise recommendation result of the target time period based on the first historical exercise data in the historical exercise data, comprises:

determining an exercise recommendation strategy for the target time period based on a physiological measurement result of the target object; and
determining the initial exercise recommendation result of the target time period based on the exercise recommendation strategy for the target time period and the first historical exercise data.

11. The method of claim 10, wherein the initial exercise recommendation result comprises an initial recommended exercise amount; and
the exercise recommendation strategy comprises increasing the exercise amount, decreasing the exercise amount, or maintaining the exercise amount.

12. The method of any one of claims 7 to 11, wherein performing adjustment processing on the initial exercise recommendation result of the target time period based on the at least part of the historical sleep data, to obtain the target exercise recommendation result of the target time period for the target object, comprises:

obtaining, based on the at least part of the historical sleep data, a sleep quality assessment result for the target object in at least one day close to the target time period; and
performing adjustment processing on the initial exercise recommendation result of the target time period based on the sleep quality assessment result for the target object in the at least one day, to obtain the target exercise recommendation result of the target time period.

13. The method of any one of claims 7 to 12, wherein performing adjustment processing on the initial exercise recommendation result of the target time period based on the at least part of the historical sleep data, to obtain the target exercise recommendation result of the target time period for the target object, comprises:
performing adjustment processing on the initial exercise recommendation result of the target time period based on the at least part of the historical sleep data and second historical exercise data in the historical exercise data, to obtain the target exercise recommendation result of the target time period, wherein the second historical exercise data comprises exercise data during at least one day within a current exercise recommendation cycle to which the target time period belongs and prior to the target time period.

14. The method of any one of claims 1 to 6, wherein obtaining the target exercise recommendation result of the target time period for the target object based on the historical sleep data and the historical exercise data, comprises:

   determining an initial exercise recommendation result of the target time period; and
   performing adjustment processing on the initial exercise recommendation result of the target time period based on at least part of the historical sleep data and second historical exercise data in the historical exercise data, to obtain the target exercise recommendation result of the target time period, wherein the at least part of the historical sleep data comprises sleep data during at least one day close to the target time period, and the second historical exercise data comprises exercise data during the at least one day close to the target time period.

15. The method of claim 13 or 14, wherein performing adjustment processing on the initial exercise recommendation result of the target time period based on the at least part of the historical sleep data and the second historical exercise data in the historical exercise data, to obtain the target exercise recommendation result of the target time period, comprises:

   obtaining an exercise assessment result for the target object based on the second historical exercise data, the exercise assessment result indicating an exercise accomplishment condition of the target object during at least one day close to the target time period;
   obtaining a sleep quality assessment result for the target object based on the at least part of the historical sleep data; and
   performing adjustment processing on the initial exercise recommendation result of the target time period based on the exercise assessment result and the sleep quality assessment result, to obtain the target exercise recommendation result of the target time period.

16. The method of claim 15, wherein performing adjustment processing on the initial exercise recommendation result of the target time period based on the exercise assessment result and the sleep quality assessment result, to obtain the target exercise recommendation result of the target time period, comprises:

   adjusting, in response to the exercise assessment result indicating that an expected exercise amount is not achieved and the sleep quality assessment result indicating that a sleep quality is lower than a preset quality index threshold, an initial recommended exercise amount comprised in the initial exercise recommendation result to a minimum exercise amount of the target object to obtain the target recommended exercise amount; or
   determining, in response to the exercise assessment result indicating that the expected exercise amount is not achieved and the sleep quality assessment result indicating that the sleep quality reaches the preset quality index threshold, the initial recommended exercise amount as the target recommended exercise amount.

17. The method of claim 15 or 16, wherein the sleep quality assessment result comprises a sleep recovery index; and obtaining the sleep quality assessment result for the target object based on the at least part of the historical sleep data, comprises:

   obtaining sleep heart rate variability (HRV) data of the target object based on the at least part of the historical sleep data; and
   obtaining the sleep recovery index of the target object based on the sleep HRV data of the target object.

18. The method of claim 17, wherein the sleep HRV data of the target object comprises at least one of a short-term sleep HRV parameter of the target object, a long-term sleep HRV parameter of the target object, or a sleep HRV parameter of the target object during a previous day of the target time period.

19. A method for sleep recommendation, comprising:

acquiring, by a terminal device, historical sleep data of a target object;

obtaining, based on at least one of attribute information of the target object or expected sleep information of the target object, a target value of at least one sleep parameter of the target object;

obtaining a sleep recommendation result of a target time period for the target object based on the target value of the at least one sleep parameter and the historical sleep data of the target object, the sleep recommendation result comprising at least one of a recommended sleep start time, a recommended wakeup time, or a recommended sleep time duration; and

outputting information about the sleep recommendation result.

20. The method of claim 19, wherein the expected sleep information of the target object comprises an expected value of the at least one sleep parameter; and

obtaining, based on at least one of the attribute information of the target object or the expected sleep information of the target object, the target value of the at least one sleep parameter of the target object, comprises:

determining, in response to the expected sleep information of the target object comprising a valid expected value of a first sleep parameter, the valid expected value of the first sleep parameter as a target value of the first sleep parameter; or

determining, in response to the expected sleep information of the target object not comprising a valid expected value of a second sleep parameter, a target value of the second sleep parameter based on the attribute information of the target object.

21. The method of claim 19 or 20, wherein obtaining the sleep recommendation result of the target time period for the target object based on the target value of the at least one sleep parameter and the historical sleep data of the target object, comprises:

determining a sleep adjustment strategy for the target object based on the historical sleep data of the target object and the target value of the at least one sleep parameter; and

obtaining the sleep recommendation result of the target time period for the target object based on the sleep adjustment strategy.

22. The method of any one of claims 19 to 21, wherein obtaining the sleep recommendation result of the target time period for the target object based on the target value of the at least one sleep parameter and the historical sleep data of the target object, comprises:

processing the historical sleep data of the target object to obtain a current value of the at least one sleep parameter of the target object; and

obtaining, in response to a difference between the current value of the at least one sleep parameter and the target value of the at least one sleep parameter exceeding a preset difference range, the sleep recommendation result of the target time period for the target object by using a progressive adjustment strategy.

23. The method of any one of claims 19 to 22, wherein obtaining the sleep recommendation result of the target time period for the target object based on the target value of the at least one sleep parameter and the historical sleep data of the target object, comprises:

obtaining, in response to a difference between a current value of the at least one sleep parameter indicated by the historical sleep data of the target object and the target value of the at least one sleep parameter exceeding a preset difference range, the sleep recommendation result of the target time period for the target object based on an adjustment step size and the current value of the at least one sleep parameter; and/or

determining, in response to the difference between the current value of the at least one sleep parameter indicated by the historical sleep data of the target object and the target value of the at least one sleep parameter being within the preset difference range, that the sleep recommendation result of the target time period for the target object comprises the target value of the at least one sleep parameter.

24. The method of any one of claims 19 to 23, further comprising:

obtaining a supplemental sleep recommendation result of the target time period for the target object based at least in part on regular sleep data of the target object during a previous day of the target time period; and/or

outputting a warning for time duration of supplemental sleep based at least in part on supplemental sleep data of the target object during the previous day of the target time period.

25. The method of claim 24, wherein obtaining the supplemental sleep recommendation result of the target time period for the target object based at least in part on the regular sleep data of the target object during the previous day of the target time period, comprises:

determining, in response to the regular sleep data of the target object during the previous day of the target time period indicating that a time duration of a regular sleep of the target object during the previous day does not reach a preset sleep time duration, that the supplemental sleep recommendation result of the target time period for the target object comprises a recommended time duration of supplemental sleep being a first time duration greater than zero; and/or
determining, in response to the regular sleep data of the target object during the previous day of the target time period indicating that the time duration of the regular sleep of the target object during the previous day reaches the preset sleep time duration, that the supplemental sleep recommendation result of the target time period for the target object comprises a recommended time duration of supplemental sleep being zero.

26. The method of claim 24 or 25, wherein outputting the warning for time duration of supplemental sleep based at least in part on the supplemental sleep data of the target object during the previous day of the target time period, comprises: outputting the warning for time duration of supplemental sleep in response to the supplemental sleep data of the target object during the previous day of the target time period indicating that a time duration of a supplemental sleep of the target object during the previous day exceeds a recommended time duration range, the warning for time duration of supplemental sleep being used for prompting to control a time duration of a supplemental sleep in the target time period to be within the recommended time duration range.

27. An electronic device, comprising:

at least one processor; and
a memory communicatively coupled to the at least one processor,
wherein the memory stores instructions executable by the at least one processor, and execution of the instructions by the at least one processor enables the at least one processor to perform the method for exercise recommendation of any one of claims 1 to 18, or to perform the method for sleep recommendation of any one of claims 19 to 26.

28. A computer-readable storage medium, configured to store instructions, wherein the instructions, when executed, cause the method for exercise recommendation of any one of claims 1 to 18 to be implemented, or cause the method for sleep recommendation of any one of claims 19 to 26 to be implemented.

29. An apparatus for exercise recommendation, comprising at least one module configured to perform the procedures in the method for exercise recommendation of any one of claims 1 to 18.

30. An apparatus for sleep recommendation, comprising at least one module configured to perform the procedures in the method for sleep recommendation of any one of claims 19 to 26.

102

108

Wearable device

Sensor

110

100

Program

106

118

Application

Intermediate device

116

Network

104

112

Server program

114

Database

Server device

FIG. 1

Acquire, by a terminal device, historical sleep data and historical exercise data of a target object — S201

Obtain, according to the historical sleep data and the historical exercise data, a target exercise recommendation result of the target time period for the target object — S202

Output information about the target exercise recommendation result — S203

FIG. 2

Acquire, by a terminal device, historical sleep data and historical exercise data of a target object ⟋⎯ S301

Determine a candidate exercise time set according to at least part of historical exercise time data included in the historical exercise data ⟋⎯ S302

Determine the target recommended exercise time from the candidate exercise time set according to the historical sleep data ⟋⎯ S303

Output information about the target exercise recommendation result ⟋⎯ S304

FIG. 3

Acquire, by a terminal device, historical sleep data and historical exercise data of a target object ⟋⎯ S401

Determine an initial exercise recommendation result of a target time period according to first historical exercise data in the historical exercise data ⟋⎯ S402

Adjust the initial exercise recommendation result according to at least part of the historical sleep data, to obtain the target exercise recommendation result ⟋⎯ S403

Output information about the target exercise recommendation result ⟋⎯ S404

FIG. 4

S501 Exercise monitoring → S503 Initial recommended exercise amount

S504 Exercise amount accomplishment frequency → S506 Dynamic adjustment strategy → Target recommended exercise amount

S502 Sleep monitoring → S505 Sleep recovery index

FIG. 5

Exercise monitoring — S601

Candidate exercise amount — S603

Sleep recovery index — S604

Target exercise amount — S607

Sleep monitoring — S602

Sleep start time — S605

Exercise recommendation — S609

Candidate exercise time — S606

Target exercise time — S608

FIG. 6

Acquire, by a terminal device, historical sleep data of a target object — S701

Obtain, according to at least one of attribute information of the target object or expected sleep information of the target object, a target value of at least one sleep parameter of the target object — S702

Obtain a sleep recommendation result of the target time period for the target object based on the target value of the at least one sleep parameter of the target object and the historical sleep data of the target object — S703

Output information about the sleep recommendation result — S704

FIG. 7

Historical sleep data — S805

Attribute information of a target object — S801

Reference sleep time duration of a group to which the target object belongs — S802

Sleep goal — S804

Step-by-step adjustment strategy — S806

Sleep recommendation — S807

Recommended sleep start time

Recommended wakeup time

Recommended sleep time duration

Subjective expectation of the target object — S803

FIG. 8

FIG. 9

FIG. 10

FIG. 11

FIG. 12

Sleep recommendation apparatus

Acquisition module — 1301    First processing module — 1302    — 1300

Second processing module — 1303    First output module — 1304

FIG. 13

Sleep recommendation apparatus

Acquisition module — 1401    First processing module — 1402    — 1400

Second processing module — 1403    First output module — 1404

Third processing module — 1405    Second output module — 1406

FIG. 14

1500

Computing unit — 1501    ROM — 1502    RAM — 1503

1504

1505

I/O interface

Input unit — 1506    Output unit — 1507    Storage unit — 1508    Communication unit — 1509

FIG. 15

## INTERNATIONAL SEARCH REPORT

| International application No. |
| --- |
| **PCT/CN2023/118017** |

| | |
| --- | --- |
| **A.** | **CLASSIFICATION OF SUBJECT MATTER** |
| | G16H20/30(2018.01)i; G16H50/30(2018.01)n; G16H70/20(2018.01)n; G06F16/9535(2019.01)n |

According to International Patent Classification (IPC) or to both national classification and IPC

| | |
| --- | --- |
| **B.** | **FIELDS SEARCHED** |

Minimum documentation searched (classification system followed by classification symbols)

IPC: G16H20/-; G16H50/-; G16H70/-; G06F16/-

Documentation searched other than minimum documentation to the extent that such documents are included in the fields searched

Electronic data base consulted during the international search (name of data base and, where practicable, search terms used)

CNTXT; ENTXT; ENTXTC; DWPI; CNKI: 运动, 推荐, 睡眠, 历史, 时间, 目标, 数据, 习惯, motion, recommendation, sleep, historical, time, target, data, habit

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| PX | CN 115701877 A (ANHUI HUAMI HEALTH TECHNOLOGY CO., LTD.) 14 February 2023 (2023-02-14) claims 1-24, and description, paragraphs [0109]-[0419] | 1-30 |
| Y | CN 109545321 A (VIVO MOBILE COMMUNICATION CO., LTD.) 29 March 2019 (2019-03-29) claims 1-13, and description, paragraphs [0017]-[0113] | 1-18, 27-29 |
| Y | CN 113870978 A (PING AN INTERNATIONAL SMART CITY TECHNOLOGY CO., LTD.) 31 December 2021 (2021-12-31) claims 1-10, and description, paragraphs [0025]-[0145] | 1-18, 27-29 |
| Y | CN 111568380 A (BEIJING SHUNYUAN KAIHUA TECHNOLOGY CO., LTD.) 25 August 2020 (2020-08-25) claims 1-16, and description, paragraphs [0038]-[0102] | 19-28, 30 |
| Y | CN 114255848 A (GREE ELECTRIC APPLIANCES, INC. OF ZHUHAI et al.) 29 March 2022 (2022-03-29) claims 1-10, and description, paragraphs [0041]-[0149] | 19-28, 30 |

| ✓ Further documents are listed in the continuation of Box C. | ✓ See patent family annex. |
| --- | --- |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **19 October 2023** | **26 December 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/118017** |

**C.     DOCUMENTS CONSIDERED TO BE RELEVANT**

| Category* | Citation of document, with indication, where appropriate, of the relevant passages | Relevant to claim No. |
| --- | --- | --- |
| A | US 2022031233 A1 (SAMSUNG ELECTRONICS CO., LTD.) 03 February 2022 (2022-02-03)<br>        entire document | 1-30 |

**INTERNATIONAL SEARCH REPORT**
Information on patent family members

International application No.

**PCT/CN2023/118017**

| Patent document cited in search report | | | Publication date (day/month/year) | Patent family member(s) | | | Publication date (day/month/year) |
|---|---|---|---|---|---|---|---|
| CN | 115701877 | A | 14 February 2023 | None | | | |
| CN | 109545321 | A | 29 March 2019 | None | | | |
| CN | 113870978 | A | 31 December 2021 | None | | | |
| CN | 111568380 | A | 25 August 2020 | CN | 111568380 | B | 18 July 2023 |
| CN | 114255848 | A | 29 March 2022 | None | | | |
| US | 2022031233 | A1 | 03 February 2022 | KR | 20220015835 | A | 08 February 2022 |
| | | | | WO | 2022025678 | A1 | 03 February 2022 |
| | | | | EP | 4142581 | A1 | 08 March 2023 |

Form PCT/ISA/210 (patent family annex) (July 2022)